# FASCICULE DE BREVET EUROPEEN

(11) **EP 2 668 293 B1**
(45) Date de publication et mention de la délivrance du brevet: **03.05.2017**
(21) Numéro de dépôt: 12706634.8
(22) Date de dépôt: 27.01.2012
(51) Int. Cl.: C12Q 1/68

(54) **PROCEDE ET KIT POUR DETERMINER IN VITRO LE STATUT IMMUNITAIRE D'UN INDIVIDU**
VERFAHREN UND KIT ZUM IN-VITRO-NACHWEIS DES IMMUNSTATUS EINER PERSON
METHOD AND KIT FOR DETERMINING, IN VITRO, THE IMMUNITY STATUS OF A PERSON

(30) Priorité: 27.01.2011 FR 1150646
(43) Date de publication de la demande: 04.12.2013
(73) Titulaire: Biomérieux, 69280 Marcy L'etoile (FR); Hospices Civils de Lyon, 69002 Lyon (FR)
(72) Inventeur: DAVIN, Fanny, 69440 Saint-Laurent d'Agny (FR); LEPAPE, Alain, F-69230 Saint-Genis Laval (FR); MONNERET, Guillaume, F-69003 Lyon (FR); FRAGER, Florence, F-69006 Lyon (FR)
(74) Mandataire: Bitaud, Valérie Marie-Odile
(86) Numéro de dépôt international: PCT/FR2012/050172
(87) Numéro de publication internationale: WO 2012/101387

(56) Documents cités:
- WO-A1-2010/049818
- WO-A1-2010/082004
- WO-A2-03/084388
- FR-A1- 2 855 832
- "GeneChip Human Genome Arrays", Affymetrix INTERNET CITATION, 2004, XP002384937, Extrait de l'Internet: URL:http://www.affymetrix.com/support/tech nical/datasheets/human_datashee t.pdf#search=%22GENECHIP%20HUMAN%20GENOME% 20ARRAYS%22 [extrait le 2006-06-12]
- GRISANTI L A ET AL: "Pro-inflammatory responses in human monocytes are <2>1-adrenergic receptor subtype dependent", MOLECULAR IMMUNOLOGY, vol. 47, no. 6, 1 mars 2010 (2010-03-01), pages 1244-1254, XP026920607, PERGAMON, GB ISSN: 0161-5890, DOI: 10.1016/J.MOLIMM.2009.12.013 [extrait le 2010-01-29]
- KENNETH MALCOLM ET AL: "Microarray analysis of lipopolysaccharide-treated human neutrophils.", AMERICAN JOURNAL OF PHYSIOLOGY. LUNG CELLULAR AND MOLECULAR PHYSIOLOGY, vol. 284, no. 4, 1 avril 2003 (2003-04-01) , pages L663-L670, XP055004693, ISSN: 1040-0605, DOI: 10.1152/ajplung.00094.2002
- HEPER Y ET AL: "Evaluation of serum C-reactive protein, procalcitonin, tumor necrosis factor alpha, and interleukin-10 levels as diagnostic and prognostic parameters in patients with community-acquired sepsis, severe sepsis, and septic shock", EUROPEAN JOURNAL OF CLINICAL MICROBIOLOGY & INFECTIOUS DISEASES, vol. 25, no. 8, 29 juillet 2006 (2006-07-29), pages 481-491, XP019386767, SPRINGER, BERLIN, DE ISSN: 1435-4373, DOI: 10.1007/S10096-006-0168-1
- MONNERET G ET AL: "The anti-inflammatory response dominates after septic shock: association of low monocyte HLA-DR expression and high interleukin-10 concentration", IMMUNOLOGY LETTERS, vol. 95, no. 2, 1 septembre 2004 (2004-09-01), pages 193-198, XP004573836, ELSEVIER BV, NL ISSN: 0165-2478, DOI: 10.1016/J.IMLET.2004.07.009
- KEEL MARIUS ET AL: "Interleukin-10 counter-regulates proinflammatory cytokine-induced inhibition of neutrophil apoptosis during severe sepsis", BLOOD, vol. 90, no. 9, 1997, pages 3356-3363, XP002656799, ISSN: 0006-4971
- BOZZA FERNANDO A ET AL: "Cytokine profiles as markers of disease severity in sepsis: a multiplex analysis.", CRITICAL CARE, vol. 11, no. 2, R49, 2007, pages 1-8, XP002656800, (LONDON, ENGLAND) ISSN: 1466-609X
- KEUN-WOOK LEE ET AL: "Direct role of NF-[kappa]B activation in Toll-like receptor-triggered HLA-DRA expression", EUROPEAN JOURNAL OF IMMUNOLOGY, vol. 36, no. 5, 1 mai 2006 (2006-05-01), pages 1254-1266, XP055004678, ISSN: 0014-2980, DOI: 10.1002/eji.200535577
- CAILLE VINCENT ET AL: "Histocompatibility leukocyte antigen-D related expression is specifically altered and predicts mortality in septic shock but not in other causes of shock", SHOCK (PHILADELPHIA): INJURY, INFLAMMATION, AND SEPSIS: LABORATORY AND CLINICAL APPROACHES, vol. 22, no. 6, 1 décembre 2004 (2004-12-01), pages 521-526, XP002488318, LIPPINCOTT WILLIAMS & WILKINS, US ISSN: 1073-2322, DOI: 10.1097/01.SHK.0000143410.63698.57
- DIDIER PAYEN ET AL: "Gene profiling in human blood leucocytes during recovery from septic shock", INTENSIVE CARE MEDICINE, vol. 34, no. 8, 5 avril 2008 (2008-04-05), pages 1371-1376, XP019619034, SPRINGER, BERLIN, DE ISSN: 1432-1238
- M. A. D. VAN ZOELEN ET AL: "Expression and Role of Myeloid-related Protein-14 in Clinical and Experimental Sepsis", AMERICAN JOURNAL OF RESPIRATORY AND CRITICAL CARE MEDICINE, vol. 180, no. 11, 1 décembre 2009 (2009-12-01), pages 1098-1106, XP055004667, ISSN: 1073-449X, DOI: 10.1164/rccm.200810-1552OC
- VOGL T ET AL: "Mrp8 and Mrp14 are endogenous activators of Toll-like receptor 4, promoting lethal, endotoxin-induced shock", NATURE MEDICINE, vol. 13, no. 9, 1 septembre 2007 (2007-09-01), pages 1042-1049, XP002568723, NATURE PUBLISHING GROUP, NEW YORK, NY, US ISSN: 1078-8956, DOI: 10.1038/NM1638 [extrait le 2007-09-02]
- Y. LE TULZO: "Monocyte Human Leukocyte Antigen-DR Transcriptional Downregulation by Cortisol during Septic Shock", AMERICAN JOURNAL OF RESPIRATORY AND CRITICAL CARE MEDICINE, vol. 169, no. 10, 15 mai 2004 (2004-05-15) , pages 1144-1151, XP055004637, ISSN: 1073-449X, DOI: 10.1164/rccm.200309-1329OC
- K. WOLK ET AL: "Multiple Mechanisms of Reduced Major Histocompatibility Complex Class II Expression in Endotoxin Tolerance", JOURNAL OF BIOLOGICAL CHEMISTRY, vol. 278, no. 20, 9 mai 2003 (2003-05-09), pages 18030-18036, XP055004620, ISSN: 0021-9258, DOI: 10.1074/jbc.M207714200
- DIVANOVIC SENAD ET AL: "Negative regulation of Toll-like receptor 4 signaling by the Toll-like receptor homolog RP105", NATURE IMMUNOLOGY, vol. 6, no. 6, 1 juin 2005 (2005-06-01), pages 571-578, XP002379423, NATURE PUBLISHING GROUP, GB ISSN: 1529-2908, DOI: 10.1038/NI1198
- OGATA HIROTAKA ET AL: "The toll-like receptor protein RP105 regulates lipopolysaccharide signaling in B cells", THE JOURNAL OF EXPERIMENTAL MEDICINE, vol. 192, no. 1, 3 juillet 2000 (2000-07-03), pages 23-29, XP002379422, ROCKEFELLER UNIVERSITY PRESS, US ISSN: 0022-1007, DOI: 10.1084/JEM.192.1.23
- J. C. DENG: "Sepsis-induced suppression of lung innate immunity is mediated by IRAK-M", JOURNAL OF CLINICAL INVESTIGATION, 17 août 2006 (2006-08-17), XP055004592, ISSN: 0021-9738, DOI: 10.1172/JCI28054
- VAN 'T VEER CORNELIS ET AL: "Induction of IRAK-M is associated with lipopolysaccharide tolerance in a human endotoxemia model", JOURNAL OF IMMUNOLOGY, vol. 179, no. 10, novembre 2007 (2007-11), pages 7110-7120, XP002656801, ISSN: 0022-1767
- LYNN VERSTREPEN ET AL: "Expression of the NF-[kappa]B inhibitor ABIN-3 in response to TNF and toll-like receptor 4 stimulation is itself regulated by NF-[kappa]B", JOURNAL OF CELLULAR AND MOLECULAR MEDICINE, vol. 12, no. 1, 23 octobre 2007 (2007-10-23), pages 316-329, XP055004569, ISSN: 1582-1838, DOI: 10.1111/j.1582-4934.2007.00187.x
- A. WULLAERT ET AL: "LIND/ABIN-3 Is a Novel Lipopolysaccharide-inducible Inhibitor of NF- B Activation", JOURNAL OF BIOLOGICAL CHEMISTRY, vol. 282, no. 1, 13 novembre 2006 (2006-11-13), pages 81-90, XP055004566, ISSN: 0021-9258, DOI: 10.1074/jbc.M607481200
- CHERON AURELIE ET AL: "Lack of recovery in monocyte human leukocyte antigen-DR expression is independently associated with the development of sepsis after major trauma", CRITICAL CARE, vol. 14, no. 6, R208, 19 novembre 2010 (2010-11-19), pages 1-10, XP021091684, BIOMED CENTRAL LTD., LONDON, GB ISSN: 1364-8535, DOI: 10.1186/CC9331
- CAZALIS MARIE-ANGELIQUE ET AL: "Early modulated mRNA expression of endotoxin tolerance genes is observed at the onset of septic shock", IMFLAMMATION RESEARCH; 8TH WORLD CONGRESS ON TRAUMA, SHOCK, INFLAMMATION AND SEPSIS/23RD SIS-EUROPE CONGRESS ON SURGICAL IN, vol. 59, no. Suppl. 1, 1 mars 2010 (2010-03-01), pages S128-S129, XP008151601, BIRKHAEUSER VERLAG, BASEL, CH; MUNICH, GERMANY ISSN: 1023-3830, DOI: 10.1007/S00011-010-0170-Z
- LUBESEDER-MARTELLATO C ET AL: "GUANYLATE-BINDING PROTEIN-1 EXPRESSION IS SELECTIVELY INDUCED BY INFLAMMATORY CYTOKINES AND IS AN ACTIVATION MARKER OF ENDOTHELIAL CELLS DURING INFLAMMATORY DISEASES", AMERICAN JOURNAL OF PATHOLOGY, vol. 161, no. 5, 1 novembre 2002 (2002-11-01), pages 1749-1759, XP009029105, AMERICAN SOCIETY FOR INVESTIGATIVE PATHOLOGY, US ISSN: 0002-9440
- VERMONT CLEMENTIEN L ET AL: "CC and CXC chemokine levels in children with meningococcal sepsis accurately predict mortality and disease severity", CRITICAL CARE, vol. 10, no. 1, R33, 20 février 2006 (2006-02-20), pages 1-8, XP021020868, BIOMED CENTRAL LTD., LONDON, GB ISSN: 1364-8535, DOI: 10.1186/CC4836
- D'AMBROSIO D ET AL: "Chemokine receptors in inflammation: an overview", JOURNAL OF IMMUNOLOGICAL METHODS, vol. 273, no. 1-2, 1 février 2003 (2003-02-01), pages 3-13, XP004402136, ELSEVIER SCIENCE PUBLISHERS B.V.,AMSTERDAM, NL ISSN: 0022-1759, DOI: 10.1016/S0022-1759(02)00414-3
- CUMMINGS C J ET AL: "Expression and function of the chemokine receptors CXCR1 and CXCR2 in sepsis", JOURNAL OF IMMUNOLOGY, vol. 162, no. 4, 15 février 1999 (1999-02-15), pages 2341-2346, XP002675478, US ISSN: 0022-1767
- NAKAMURA ET AL: "Treatment with polymyxin B-immobilized fiber reduces platelet activation in septic shock patients: decrease in plasma levels of soluble P-selectin, platelet factor 4 and beta-thromboglobulin.", INFLAMMATION RESEARCH, vol. 48, no. 4, 1 avril 1999 (1999-04-01), pages 171-175, XP055026445, ISSN: 1023-3830
- PUNYADEERA CHAMINDIE ET AL: "A biomarker panel to discriminate between systemic inflammatory response syndrome and sepsis and sepsis severity.", JOURNAL OF EMERGENCIES, TRAUMA, AND SHOCK , vol. 3, no. 1 janvier 2010 (2010-01), page 18PP, XP002675479, ISSN: 0974-519X Extrait de l'Internet: URL:http://www.ncbi.nlm.nih.gov/pmc/articl es/PMC2823139/?report=printable [extrait le 2012-05-08]

## Description

La présente invention concerne un procédé et un kit pour déterminer *in vitro* le statut immunitaire d'un individu.

Les syndromes septiques représentent l'une des premières causes de mortalité dans les services de réanimation. Ils peuvent résulter d'une infection bactérienne, virale, mycosique ou parasitaire. Parmi les syndromes septiques, on distingue par ordre croissant de gravité le SIRS (Systemic Inflammatory Response Syndrome), le sepsis, le sepsis sévère et le choc septique.
- le SIRS, est une réponse systémique inflammatoire déclenchée par une variété de causes infectieuses ou non. Parmi les états de SIRS déclenchés par des causes non infectieuses, on peut citer les états traumatiques, les brûlures, les pancréatites, les syndromes respiratoires aiguës. Une réponse systémique inflammatoire se manifeste par au moins deux des signes suivants : a) température supérieure à 38°C ou inférieure à 36°C ; b) rythme cardiaque supérieur à 90 battements par minute ; c) rythme respiratoire supérieur à 20 respirations par minute ; d) nombre de leucocytes supérieur à 12000/mm³ ou inférieur à 4000/mm³,
- le sepsis est un syndrome de réponse systémique inflammatoire en relation avec une infection,
- un sepsis sévère est un sepsis associé à une hypotension artérielle et/ou hypoperfusion et/ou dysfonctionnement d'au moins un organe,
- le choc septique est un sepsis sévère associé à une hypotension persistante et peut être qualifié par :
   - la présence d'un site infectieux identifié,
   - une hypotension persistante malgré des remplissages adéquats et des traitements vasopresseurs.

D'une manière générale, les signes d'un sepsis, d'un sepsis sévère et d'un choc septique sont proches, et la différence entre ces trois situations réside principalement dans l'importance de la perturbation de toutes les fonctions vitales.

Les syndromes septiques ont longtemps été considérés uniquement comme une réponse inflammatoire non régulée conduisant, en réponse à une dissémination bactérienne initiale, à une défaillance multi-viscérales de l'organisme. Cependant, les échecs des approches thérapeutiques, principalement anti-inflammatoires, expérimentées durant ces dernières années, ainsi que l'incapacité à caractériser la forte hétérogénéité des patients septiques ont contribué à remettre en cause la vision essentiellement pro-inflammatoire de la physiopathologie des syndromes septiques.

Actuellement, la réponse immunitaire au cours du choc septique est plutôt décrite en deux phases successives. Après une brève phase initiale très inflammatoire, responsable de la symptomatologie du choc proprement dit, se met en place un état de dépression immunitaire induit par les mécanismes immunosuppresseurs chargés de contrôler la réponse pro-inflammatoire. Ainsi, cette composante anti-inflammatoire de la réaction à l'infection, initialement perçue comme purement compensatrice, s'est révélée prédominante chez la majorité des malades et s'accompagne d'une immunosuppression et d'une hypo-réactivité cellulaire marquée. De plus, il semblerait que la mortalité survienne principalement à distance du choc (ou de la défaillance d'organe initiale) dans un contexte où tous les patients présentent des signes biologiques d'immunodépression. Par conséquent, deux causes de mortalité seraient donc présentes au cours du choc septique impliquant des thérapeutiques différentes. La prévention de la mortalité initiale due aux dysfonctions d'organes doit être mise en place le plus précocement possible après le diagnostic et constitue encore à ce jour l'urgence thérapeutique en service de réanimation. La prévention de la mortalité tardive, potentiellement due à l'état de reprogrammation leucocytaire, pourrait nécessiter des thérapeutiques pro-inflammatoires permettant de stimuler le système immunitaire.

La prise en compte de l'hétérogénéité des patients septiques devient donc de plus en plus incontournable à la sélection d'une population homogène lors des essais cliniques mais également lors de la mise en place de thérapeutiques de plus en plus personnalisées. Ceci est d'autant plus important que certaines thérapeutiques proposées développent des effets totalement opposés sur la réponse immunitaire et peuvent donc être délétères aux patients si ceux-ci ne présentent pas le statut immunó-inflammatoire adapté au choix thérapeutique. Il est donc important de disposer d'un procédé et d'un kit qui permettent d'établir le statut immunitaire global du patient de manière fiable quand il entre au service d'urgence ou de réanimation et qui permettent aussi de définir le niveau de dérégulation de chacune des composantes de la réponse immuno-inflammatoire pour la mise en place de la thérapeutique la mieux ciblée en fonction de la réponse immunitaire établie (innée et/ou adaptative). Le procédé et le kit précités sont également utilisables pour suivre l'évolution du statut immunitaire du patient en réponse à un traitement thérapeutique.

La demande FR 2 855 832 A2 se propose d'établir un diagnostic et/ou pronostic d'un syndrome septique à partir de l'expression d'au moins deux gènes choisis parmi plusieurs gènes cibles. Néanmoins cette demande ne décrit pas la détermination du statut immunitaire d'un individu.

L'invention a donc pour objet un procédé pour déterminer *in vitro* le statut immunitaire d'un individu selon lequel:
a. on dispose d'un échantillon sanguin de l'individu,
b. on dispose d'au moins deux réactifs spécifiques d'au moins deux produits d'expression d'au moins deux gènes cibles respectivement choisis dans au moins une des familles de gènes identifiées ci-dessous ou respectivement choisis dans deux familles de gènes différentes choisies parmi celles identifiées ci-dessous:
   - la famille des marqueurs exprimés à la surface des cellules circulantes,
   - la famille des alarmines,
   - la famille des molécules régulatrices, activatrices ou inhibitrices, des voies de signalisation inflammatoires intracellulaires,
   - la famille des facteurs de transcription,
   - la famille des marqueurs solubles pro-inflammatoires,
   - la famille des marqueurs solubles anti-inflammatoires,
   - la famille des molécules à activité anti-infectieuse,
   - la famille des chemokines,
   - la famille des molécules du cycle cellulaire, et
   - la famille des molécules du métabolisme,
c. on détermine l'expression desdits au moins deux gènes cibles,
d. on compare l'expression desdits au moins deux gènes cibles respectivement avec une expression de référence, une modification dans l'expression desdits au moins deux gènes cibles par rapport à leur expression de référence étant un indicateur d'une modification du statut immunitaire de l'individu et une corrélation entre l'expression desdits au moins deux gènes cibles avec leur expression de référence étant un indicateur d'une normalité du statut immunitaire de l'individu.

Dans des modes de réalisation particuliers de l'invention, dans l'étape b. on dispose :
- soit d'au moins trois réactifs spécifiques d'au moins trois produits d'expression d'au moins trois gènes cibles respectivement choisis dans trois familles de gènes différentes choisies parmi celles identifiées ci-dessus,
- soit d'au moins quatre réactifs spécifiques d'au moins quatre produits d'expression d'au moins quatre gènes cibles respectivement choisis dans quatre familles de gènes différentes choisies parmi les familles de gènes identifiées ci-dessus,
- soit d'au moins cinq réactifs spécifiques d'au moins cinq produits d'expression de cinq gènes cibles respectivement choisis dans les dix familles de gènes différentes choisies parmi celles identifiées ci-dessus.

Les gènes cibles préférés sont ceux identifiés ci-dessous : Les clusters de différentiation (CD) et les complexes majeurs d'histocompatibilité de classe II, tels que HLA-DR, en particulier HLA-DR alpha, et autres clusters de différentiation qui appartiennent à la famille des marqueurs cellulaires,
S100A9 et S100A8 qui appartiennent à la famille des alarmines, ABIN-3 (TNFAIP3 interacting protein 3), IRAK-M (Interleukin-1 receptor-associated kinase 3) et LY64 (encore dénommmé CD180) qui appartiennent à la famille des molécules régulatrices des récepteurs membranaires ou solubles,
CIITA qui appartient à la famille des facteurs de transcription, TNF, en particulier TNF-alpha, COX-2 (encore dénommé PTGS2), FCN1 et MMP7 qui appartiennent à la famille des marqueurs solubles pro-inflammatoires,
IL-10 qui appartient à la famille des marqueurs solubles anti-inflammatoires,
GBP-1 qui appartient à la famille des molécules à activité anti-infectieuse, CXCL-1, CXCL-5, CXCL-7 et CXCL-10 qui appartiennent à la famille des chemokines,
RHOU qui appartient à la famille des molécules du cycle cellulaire, et PID1 qui appartient à la famille des molécules du métabolisme. A noter que LY64 (encore dénommé CD180) a un rôle régulateur et de ce fait est classé dans la famille des molécules régulatrices, mais est également un récepteur de surface cellulaire.

Dans un mode de réalisation du procédé de l'invention, l'échantillon sanguin est un échantillon prélevé chez un individu ayant un traitement immunomodulateur. Le terme immunomodulateur sera défini plus en détail dans le paragraphe « Définitions ».

Le procédé de l'invention peut aussi comprendre une étape supplémentaire selon laquelle dans l'étape a. on extrait les cellules mononucléees du sang périphérique (PBMCs) de l'échantillon sanguin et on réalise les étapes subséquentes b. à d. sur lesdites cellules mononucléees sanguines, comme décrit précédemment.

Les réactifs spécifiques des produits d'expression des gènes cibles décrits ci-dessus comprennent :
- soit au moins une amorce d'amplification spécifique desdits gènes cibles,
- soit au moins une sonde d'hybridation spécifique desdits gènes cibles,
- soit au moins un anticorps.

L'invention a aussi pour objet un kit pour déterminer *in vitro* le statut immunitaire d'un individu comprenant au moins deux réactifs spécifiques d'au moins deux produits d'expression d'au moins deux gènes cibles respectivement choisis dans au moins une des familles de gènes identifiées ci-dessous ou respectivement choisis dans deux familles de gènes différentes choisies parmi celles identifiées ci-dessous:
- la famille des marqueurs exprimés à la surface des cellules circulantes,
- la famille des alarmines,
- la famille des molécules régulatrices, activatrices ou inhibitrices, des voies de signalisation inflammatoires intracellulaires,
- la famille des facteurs de transcription,
- la famille des marqueurs solubles pro-inflammatoires,
- la famille des marqueurs solubles anti-inflammatoires,
- la famille des molécules à activité anti-infectieuse,
- la famille des chemokines,
- la famille des molécules du cycle cellulaire, et
- la famille des molécules du métabolisme.

Dans des modes de réalisation particuliers de l'invention le kit comprend :
- soit au moins trois réactifs spécifiques d'au moins trois produits d'expression de trois gènes cibles appartenant respectivement à trois familles de gènes cibles différentes, répondant aux définitions ci-dessus,
- soit au moins quatre réactifs spécifiques d'au moins quatre produits d'expression de quatre gènes cibles appartenant respectivement à quatre familles de gènes cibles différentes telles que définies précédemment,
- soit au moins cinq réactifs spécifiques d'au moins cinq produits d'expression de cinq gènes cibles appartenant respectivement à cinq familles de gènes cibles différentes telles que définies ci-dessus.

Les gènes cibles préférés sont ceux identifiés précédemment, à savoir :
HLA-DR, en particulier HLA-DR alpha, et CD qui appartiennent à la famille des marqueurs cellulaires,
S100A9 et S100A8 qui appartiennent à la famille des alarmines, ABIN-3, IRAK-M et LY64 (encore dénommé CD180) qui appartiennent à la famille des molécules régulatrices des récepteurs membranaires ou solubles,
CIITA qui appartient à la famille des facteurs de transcription, TNF, en particulier TNF-alpha, COX-2 (encore dénommé PTGS2), FCN1 et MMP7 qui appartiennent à la famille des marqueurs solubles pro-inflammatoires, IL-10 qui appartient à la famille des marqueurs solubles anti-inflammatoires,
GBP-1 qui appartient à la famille des molécules à activité anti-infectieuse,
CXCL-1, CXCL-5, CXCL-7 et CXCL-10 qui appartiennent à la famille des chemokines,
RHOU qui appartient à la famille des molécules du cycle cellulaire,
et PID1 qui appartient à la famille des molécules du métabolisme.

Comme précisé ci-dessus, les réactifs spécifiques des produits d'expression des gènes cibles comprennent :
- soit au moins une amorce d'amplification spécifique desdits gènes cibles,
- soit au moins une sonde d'hybridation spécifique desdits gènes cibles,
- soit au moins un anticorps.

L'invention a encore pour objet l'utilisation d'au moins deux réactifs spécifiques d'au moins deux produits d'expression d'au moins deux gènes cibles respectivement choisis dans au moins une des familles de gènes suivantes ou respectivement choisis dans deux des familles de gènes suivantes:
- la famille des marqueurs exprimés à la surface des cellules circulantes,
- la famille des alarmines,
- la famille des molécules régulatrices, activatrices ou inhibitrices, des voies de signalisation inflammatoires intracellulaires,

- la famille des facteurs de transcription,
- la famille des marqueurs solubles pro-inflammatoires,
- la famille des marqueurs solubles anti-inflammatoires,
- la famille des molécules à activité anti-infectieuse,
- la famille des chemokines,
- la famille des molécules du cycle cellulaire, et
- la famille des molécules du métabolisme.

Dans des modes de réalisation particuliers de l'invention on utilise :
- soit au moins trois réactifs spécifiques d'au moins trois produits d'expression de trois gènes cibles appartenant respectivement à trois familles de gènes cibles différentes, répondant aux définitions ci-dessus,
- soit au moins quatre réactifs spécifiques d'au moins quatre produits d'expression de quatre gènes cibles appartenant respectivement à quatre familles de gènes cibles différentes telles que définies précédemment,
- soit au moins cinq réactifs spécifiques d'au moins cinq produits d'expression de cinq gènes cibles appartenant respectivement aux cinq familles de gènes différentes définies ci-dessus.

Les gènes cibles préférés et les réactifs spécifiques répondent respectivement aux définitions données ci-dessus pour le procédé ou le kit de l'invention.

### Définitions

Echantillon de sang ou échantillon sanguin signifie indifféremment le sang total, le sérum ou le plasma.

On entend par produit d'expression d'un gène spécifique cible, l'ARN messager ou un fragment d'ARNm, l'ADNc ou un fragment d'ADNc, une protéine ou un fragment de protéine.

On entend par réactif spécifique un réactif qui réagit avec un matériel biologique de l'échantillon de sang ou des cellules mononucléées du sang périphérique (PBMCs) afin de mettre en évidence, d'une manière directe ou indirecte l'expression d'un gène cible, qui peut être déterminé par l'analyse des ARNm issus de ce gène, ou par l'analyse de la protéine codée par ce gène.

A titre indicatif, lorsque l'on souhaite déterminer l'expression d'un gène cible par l'analyse de la protéine codée par ce gène, ce réactif spécifique comprend au moins un anticorps spécifique de la protéine codée par ce gène cible.

A titre indicatif, lorsque l'on souhaite déterminer l'expression d'un gène cible par l'analyse des ARNm transcrits à partir de ce gène, ce réactif spécifique comprend au moins une amorce d'amplification spécifique de l'ADN complémentaire de cet ARNm (on parle alors d'amorce d'amplification spécifique d'un gène cible). L'ADN complémentaire d'un ARNm peut être obtenu selon un protocole bien connu de l'homme du métier. Par exememple, on extrait les ARN totaux (comprenant les ARN ribosomaux, les ARN de transfert, les ARNm) de l'échantillon sanguin ou des PBMCs. On réalise ensuite une réaction de transcription inverse à l'aide d'une enzyme transcriptase inverse qui permet d'obtenir, à partir d'un fragment d'ARN, un fragment complémentaire d'ADN (ADNc). La réalisation d'une telle étape est bien connue de l'homme du métier. Lorsque l'on souhaite plus particulièrement obtenir uniquement les ADN complémentaires des ARN messagers, on réalise cette étape enzymatique en présence de fragments nucléotidiques comprenant uniquement des bases thymine (polyT), qui s'hybrident par complémentarité sur la séquence polyA des différents ARNm afin de former un complexe polyT-polyA qui sert alors de point de départ à la réaction de transcription inverse réalisée par l'enzyme transcriptase inverse. On obtient alors différents ADN complémentaires des différents ARN messagers initialement présents dans l'échantillon sanguin ou dans les PBMCs. Dans la suite de l'exposé, on désigne par ADNc, un ADN complémentaire d'un ARN messager.

Par amorce d'amplification, on entend un fragment nucléotidique comprenant de 5 à 100 motifs nucléotidiques, préférentiellement de 15 à 25 motifs nucléotidiques et possédant une spécificité d'hybridation dans des conditions déterminées pour l'initiation d'une polymérisation enzymatique, par exemple dans une réaction d'amplification enzymatique.

Par réaction d'amplification enzymatique, on entend un processus générant de multiples copies d'un fragment nucléotidique cible à l'aide d'amorces d'amplification spécifiques par l'action d'au moins une enzyme. De telles réactions d'amplification sont bien connues de l'homme du métier et on peut citer notamment les techniques suivantes : PCR (Polymerase Chain Reaction), LCR (Ligase Chain Reaction), RCR (Repair Chain Reaction), 3SR (Self Sustained Sequence Replication) avec la demande de brevet WO-A-90/06995, NASBA (Nucleic Acid Sequence-Based Amplification), et TMA (Transcription Mediated Amplification) avec le brevet US-A-5,399,491.
On parle alors d'amplicons pour désigner les polynucléotides générés par une technique d'amplification enzymatique. Préférentiellement, lorsque l'amplification enzymatique est une PCR, le réactif spécifique comprend au moins 2 amorces d'amplification spécifiques afin d'amplifier une région particulière de l'ADN complémentaire de l'ARNm issu du gène cible. Lorsque l'amplification enzymatique est une PCR réalisée après une réaction de transcription inverse, on parle de RT-PCR.

Par sonde d'hybridation, on entend un fragment nucléotidique comprenant de 5 à 100 motifs nucléotidiques, notamment de 6 à 35 motifs nucléotidiques, possédant une spécificité d'hybridation dans des conditions déterminées pour former un complexe d'hybridation avec un fragment nucléotidique cible. Dans la présente invention, le fragment nucléotidique cible peut être une séquence nucléotidique comprise dans un ARN messager ou une séquence nucléotidique comprise dans un ADN complémentaire obtenu par transcription inverse dudit ARN messager.

Par hybridation, on entend le processus au cours duquel, dans des conditions appropriées, deux fragments nucléotidiques, tels que par exemple une sonde d'hybridation et un fragment nucléotidique cible, ayant des séquences suffisamment complémentaires sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier. En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.

Par détection on entend soit une détection directe par une méthode physique, soit une méthode de détection à l'aide d'un marqueur. De nombreuses méthodes de détection existent pour la détection des acides nucléiques [1,2].

Par marqueur, on entend un traceur capable d'engendrer un signal. Une liste non limitative de ces traceurs comprend les enzymes qui produisent un signal détectable par exemple par colorimétrie, fluorescence ou luminescence, comme la peroxydase de raifort, la phosphatase alcaline, la β-galactosidase, la glucose-6-phosphate déshydrogénase ; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I. Ainsi, le polynucléotide peut être marqué pendant l'étape d'amplification enzymatique, par exemple en utilisant un nucléotide triphosphate marqué pour la réaction d'amplification. Le nucléotide marqué sera un désoxyribonucléotide dans les systèmes d'amplification générant un ADN, comme la PCR, ou un ribonucléotide dans les techniques d'amplification générant un ARN, comme les techniques TMA ou NASBA. Le polynucléotide peut aussi être marqué après l'étape d'amplification, par exemple en hybridant une sonde marquée selon la technique d'hybridation sandwich décrite dans le document WO-A-91/19812.

Au sens de la présente invention, la sonde d'hybridation peut être une sonde dite de capture. Dans ce cas, le fragment nucléotidique cible peut être préalablement marqué au moyen d'un marqueur. La sonde dite de capture est immobilisée ou immobilisable sur un support solide par tout moyen approprié, c'est-à-dire directement ou indirectement, par exemple par covalence ou adsorption. On réalise alors une réaction d'hybridation entre ladite sonde de détection et le fragment nucléotidique cible marqué.

La sonde d'hybridation peut également être une sonde dite de détection. Dans ce cas, la sonde d'hybridation peut être marquée au moyen d'un marqueur. On réalise alors une réaction d'hybridation entre ladite sonde de capture et le fragment nucléotidique cible.

Que l'on utilise une sonde dite de capture ou une sonde dite de détection, la réaction d'hybridation peut être réalisée sur un support solide qui inclut tous les matériaux sur lesquels peut être immobilisé un acide nucléique. Des matériaux de synthèse ou des matériaux naturels, éventuellement modifiés chimiquement, peuvent être utilisés comme support solide, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels etc. Le support solide peut être sous la forme d'une plaque de microtitration, d'une membrane comme décrit dans la demande WO-A-94/12670, d'une particule ou d'une biopuce.

Dans la présente invention, la détermination de l'expression d'un gène cible peut être analysée par l'expression des ARNm qui sont transcrits à un temps donné. Dans ce cas, le matériel biologique est un acide nucléique, et le réactif spécifique peut être indifféremment une amorce d'amplification ou une sonde d'hybridation telles que définies précédemment.

On peut déterminer l'expression d'un gène cible de la manière suivante:
1) après avoir extrait les ARN totaux d'un échantillon sanguin ou de PBMCs, on réalise une étape de transcription inverse, telle que décrite précédemment afin d'obtenir les différents ADN complémentaires des différents ARN messagers initialement présents dans l'échantillon sanguin ou dans les PBMCs (ou ADNc)
2) on amplifie spécifiquement les ADNc. Dans ce cas, le réactif spécifique utilisé comprend au moins une amorce d'amplification spécifique du gène cible, telle que définie précédemment. Cette étape peut être réalisée notamment par une réaction d'amplification de type PCR ou par toute autre technique d'amplification appropriée
3) on détermine l'expression du gène cible en quantifiant les ADNc. Les ADNc peuvent être quantifiés notamment par l'utilisation d'une gamme de quantification obtenue par une réaction d'amplification conduite jusqu'à saturation. Afin de tenir compte de la variabilité d'efficacité enzymatique qui peut être observée lors des différentes étapes (transcription inverse, PCR quantitative...), on peut normaliser l'expression du gène cible des différents groupes de patients, par la détermination simultanée de l'expression d'un gène dit de ménage, dont l'expression est similaire chez les différents groupes de patients. En réalisant un rapport entre l'expression du gène cible et l'expression du gène de ménage, on corrige ainsi toute variabilité entre les différentes expérimentations. L'homme du métier pourra se référer notamment aux publications suivantes : [3-4] Bustin SA Journal of molecular endocrinology, 2002, 29 : 23-39 ; Giulietti A Methods, 2001, 25 : 386-401

On peut également déterminer l'expression d'un gène cible de la manière suivante:
1) après avoir extrait les ARN totaux d'un échantillon sanguin ou des PBMCs, on réalise une étape de transcription inverse, telle que décrite précédemment afin d'obtenir les différents ADN complémentaires des différents ARN messagers initialement présents dans l'échantillon ou les PBMCs (ou ADNc)
2) on immobilise les ADNc sur une membrane
3) on détermine l'expression du gène cible en hybridant les ADNc à des sondes d'hybridation spécifiques du gène cible préalablement marquées. De telles techniques d'hybridation sont bien connues de l'homme du métier, et on peut citer notamment la technique du Northern blot. Cette réaction d'hybridation peut être réalisée après une étape d'amplification spécifique des ADN complémentaires des ARN messagers d'un gène cible lorsque notamment le gène est faiblement exprimé

L'expression d'un gène cible peut être également analysée par l'expression des protéines codées par le gène cible. Dans ce cas, le matériel biologique est une protéine et plusieurs techniques de détection avec ou sans ligand peuvent être utilisées. La spectrométrie de masse peut être utilisée comme technique de détection sans ligand. Le réactif spécifique peut être un anticorps spécifique de la protéine codée par le gène cible pour un système de détection avec ligand.

Les anticorps recombinants, spécifiques de la protéine traduite du gène cible peuvent être obtenus selon des procédés classiques connus de l'homme du métier, à partir d'organismes procaryotes, tels que bactéries, ou à partir d'organismes eucaryotes, tels que levures, cellules de mammifères, de plantes, d'insectes ou d'animaux, ou par des systèmes de production extra-cellulaire.

Les anticorps monoclonaux peuvent être préparés selon les techniques classiques connues de l'homme du métier telles que la technique des hybridomes dont le principe général est rappelé ci-dessous.

Dans un premier temps, on immunise un animal, généralement une souris, (ou des cellules en culture dans le cadre d'immunisations *in vitro*) avec un antigène cible d'intérêt, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité.

Des fragments d'anticorps peuvent par exemple être obtenus par protéolyse. Ainsi, ils peuvent être obtenus par digestion enzymatique, résultant en des fragments de type Fab (traitement à la papaïne [5] ou de type F(ab)'2 (traitement à la pepsine; [6]. Ils peuvent également être préparés par voie recombinante [7]. Un autre fragment d'anticorps qui convient aux fins de l'invention comprend un fragment Fv qui est un dimère constitué de l'association non covalente du domaine variable léger (VL) et du domaine variable lourd (VH) du fragment Fab, donc de l'association de deux chaînes polypeptidiques. Afin d'améliorer la stabilité du fragment Fv due à la dissociation des deux chaînes polypeptidiques, ce fragment Fv peut être modifié par génie génétique en insérant un lien peptidique adapté entre le domaine VL et le domaine VH [8]. On parle alors de fragment scFv (« single chain Fragment variable ») car il est constitué d'une seule chaîne polypeptidique. L'utilisation d'un lien peptidique composé préférentiellement de 15 à 25 acides aminés permet de relier l'extrémité C-terminale d'un domaine à l'extrémité N-terminale de l'autre domaine, constituant ainsi une molécule monomérique dotée de propriétés de liaison similaires à celles de l'anticorps sous sa forme complète. Les deux orientations des domaines VL et VH conviennent (VL-lien-VH et VH-lien-VL) car elles présentent des propriétés fonctionnelles identiques. Bien entendu, tout fragment connu de l'homme du métier et présentant les caractéristiques immunologiques définies ci-dessus convient aux fins de l'invention.

Lorsque le matériel biologique est une protéine issue de l'expression d'un gène, on peut déterminer l'expression de ce dernier, en détectant et ou quantifiant la dite protéine par Western Blot ou ELISA, ou toute autre méthode connue de l'homme du métier, telle qu'une méthode de chimiluminescence à partir du matériel biologique.

La technique ELISA (« Enzyme Linked Immuno Sorbent Assay ») est un dosage immunoenzymatique sur support solide. Ce test entre dans le cadre plus général des EIA (« Enzyme Immunoassays ») dans lequel le dosage est couplé à une réaction catalysée par une enzyme. La technique utilise un ou deux anticorps. L'anticorps de détection de la formation de complexes immuns (antigène / anticorps) est couplé à l'enzyme et peut générer l'émission d'un signal par un substrat chromogène ou fluorogène.

La technique Western Blot est un test pour détecter une protéine spécifique dans un échantillon à l'aide d'un anticorps spécifique de cette protéine comprenant les étapes suivantes telle que décrites ci-dessous.

La première étape est une électrophorèse sur gel, qui permet de séparer les protéines de l'échantillon selon leur taille.

Les protéines dans le gel sont alors transférées sur une membrane (nitrocellulose, PVDF...) par pression ou par application d'un courant électrique, les protéines se fixant à la membrane grâce à des interactions hydrophobes et ioniques.
Après saturation des sites d'interaction non spécifique, un premier anticorps, spécifique de la protéine à étudier (anticorps primaire) est incubé avec la membrane.
La membrane est ensuite rincée afin d'enlever les anticorps primaires non liés, puis incubée avec des anticorps dits secondaires, qui vont se lier aux anticorps primaires. Cet anticorps secondaire est habituellement lié à une enzyme qui permet l'identification visuelle de la protéine étudiée sur la membrane. L'ajout d'un substrat marqué de l'enzyme engendre une réaction colorée qui est visible sur la membrane.

Par produits immunomodulateurs on entend les immunostimulants et les immunosuppresseurs.

L'immunostimulation est recherchée par exemple dans le cas d'immunosuppression (syndrome septique), d'immunodéficience (SIDA), dans le cas d'infections chroniques et dans le cas de cancers. Les cytokines peuvent être des molécules immunostimulantes. Le terme « cytokines » désigne un groupe hétérogène de protéines produites par les cellules de la lignée blanche. Elles ont un rôle essentiel dans la régulation du système immunitaire et de l'hématopoïèse. Les principales familles de molécules utilisées en thérapeutique comme immunostimulant sont :
- la famille des interleukines, telles que l'IL-2, l'IL-7 et l'IL-15,
- la famille des facteurs de croissance, tels que G-CSF (Granulocyte Colony Stimulating Factor), GM-CSF (Granulocyte Macrophage Colony Stimulating Factor) et FLT3-L (Fms-Like Tyrosine kinase 3 Ligand),
- la famille des interférons tels que les interférons α, β et γ...),
- la famille des agonistes Toll,
- la famille des anticorps bloquants (anti-IL-10, anti-CTLA4, anti-PD-1...),
- la famille des transferrines, telles que lactoferrine et talactoferrine,
- la famille des molécules bloquant l'apoptose , telles que les inhibiteurs de protéases, les inhibiteurs de caspases, les inhibiteurs de Fas/FasL, les inducteurs de Bcl-2.

L'immunosuppression est recherchée pour inhiber ou prévenir l'activité du système immunitaire. On utilise les immunosuppresseurs pour limiter les dysfonctions immunitaires engendrées par une réponse pro-inflammatoire exacerbée dans le cadre d'un syndrome septique, prévenir le rejet de greffe d'organes et de transplanté, pour traiter des maladies auto-immunes ou des maladies susceptibles d'être d'origine auto-immune. Les immunosuppresseurs sont généralement répartis en plusieurs familles :
- la famille des glucocorticoides,
- la famille des cytostatiques,
- la famille des anticorps bloquants (anti-TNF),
- la famille des molécules agissant sur les immunophilines et,
- d'autres molécules, en particulier, les cytokines qui peuvent être des molécules immunosuppressives, telles que l'IL-10, l'IL-3, TGF-β, IL-1 RA.

### FIGURES

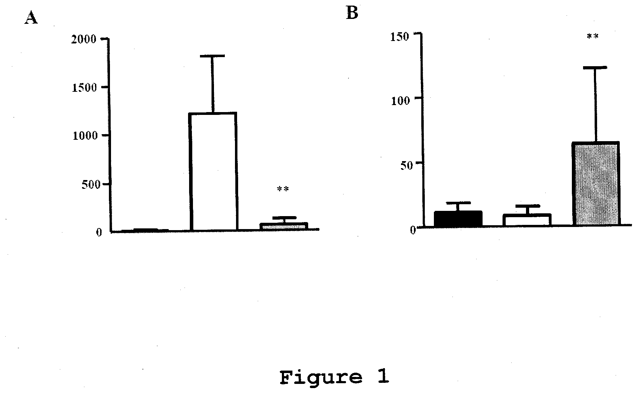

Figure 1 : Les figures 1A et 1B représentent respectivement les concentrations de TNF-α et d'IL-10 produites par des PBMCs, issues de 10 volontaires sains, stimulées par du LPS. Le dosage a été effectué par un test ELISA sur des surnageants de culture obtenus à 45 heures. L'axe des ordonnées représentent les concentrations protéiques (pg / ml) de TNF-α (figure 1A) et d'IL-10 (figure 1B). Les histogrammes noirs représentent les contrôles négatifs (sans stimulation). Les histogrammes blancs correspondent aux cellules stimulées une seule fois avec 100 ng / mL de LPS et les histogrammes gris représentent les cellules soumises à deux stimulations par du LPS (2 ng / ml puis 100 ng / ml). Les données sont représentées en moyenne +/- écart type. Un test appareillé de Wilcoxon a été réalisé pour l'analyse statistique des résultats: **, signifie que p<0,005 vs cellules stimulées une fois au LPS.

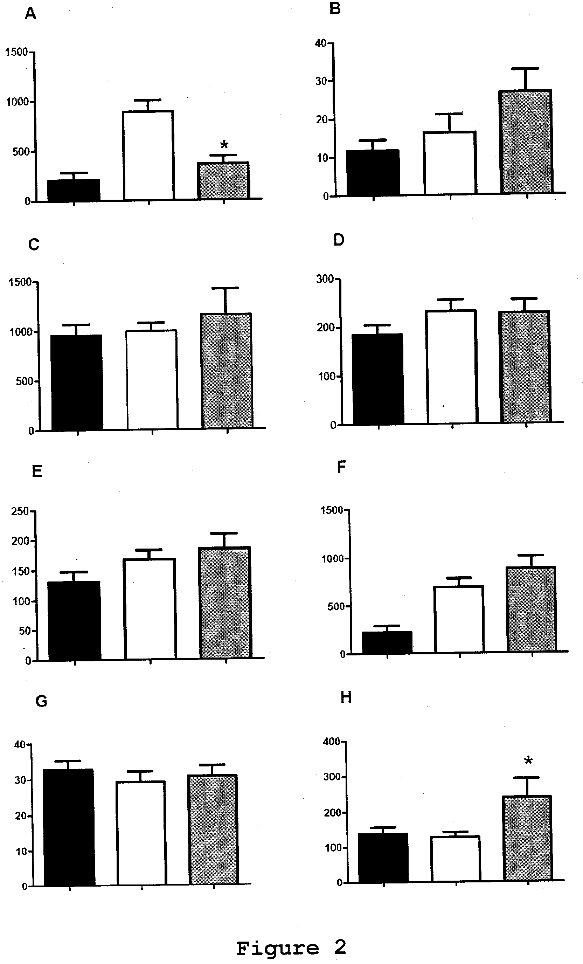

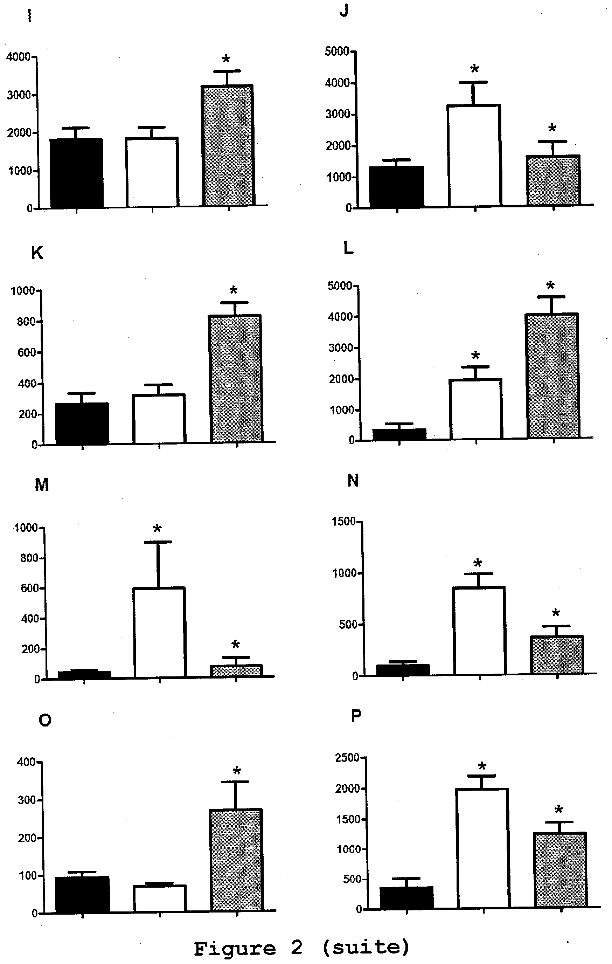

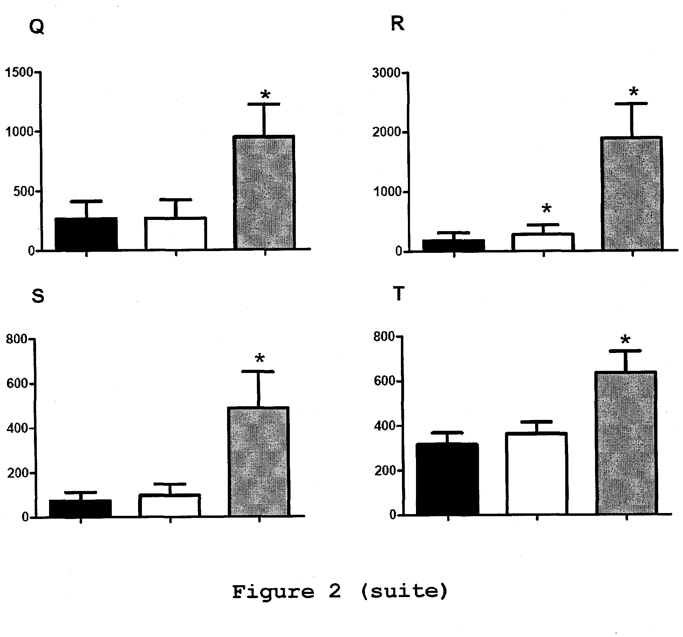

Figure 2 : La figure 2 montre l'expression des gènes de TNF alpha (A), IL-10 (B), HLA-DRA (C), CIITA (D), IRAK-M (E), ABIN-3 (F), LY64 (G), S100A9 (H), S100A8 (I), GBP1 (J), MMP7 (K), CXCL1 (L), CXCL10 (M), COX2 (N), FCN1 (O), TNFAIP6 (P), CXCL7 (Q), CXCL5 (R), PID1 (S) et RHOU (T) par des PBMCs, issues de 6 volontaires sains, stimulées par du LPS et quantifiées par biopuces après 45 heures. L'axe des ordonnées représente l'intensité de fluorescence de chacune des sondes d'hybridation : (A) 207113_s_at pour le TNF alpha ; (B) 207433_at pour l'IL-10; (C) 208894_at pour HLA-DRA ; (D) 205101_at pour CIITA; (E) 213817_at pour IRAK-M ; (F) 220655_at pour ABIN-3 ; (G) 206206_at pour LY64; (H) 203535_at pour S100A9 ; (I) 202917_s_at pour S100A8 ; (J) 202269_x_at pour GBP1 ; (K) 204259_art pour MMP7 ; (L) 204470_at pour CXCL1 ; (M) 204533_at pour CXCL10 ; (N) 204748_at pour COX2; (O) 205237_at pour FCN1; (P) 206026_s_at pour TNFAIP6; (Q) 214146_s_at pour CXCL7; (R) 215101_s_at pour CXCL5 ; (S) 219093_at pour PID1 et (T) 223168_at pour RHOU. Les histogrammes noirs représentent les contrôles négatifs (sans stimulation). Les histogrammes blancs correspondent aux cellules stimulées une seule fois avec 100 ng / mL de LPS et les histogrammes gris représentent les cellules soumises à deux stimulations par du LPS (2 ng / ml puis 100 ng/ ml). Les données sont représentées en moyenne +/- écart type. Un t-test appareillé a été réalisé pour l'analyse statistique des résultats: *, signifie que p<0,05 vs cellules stimulées une fois au LPS.

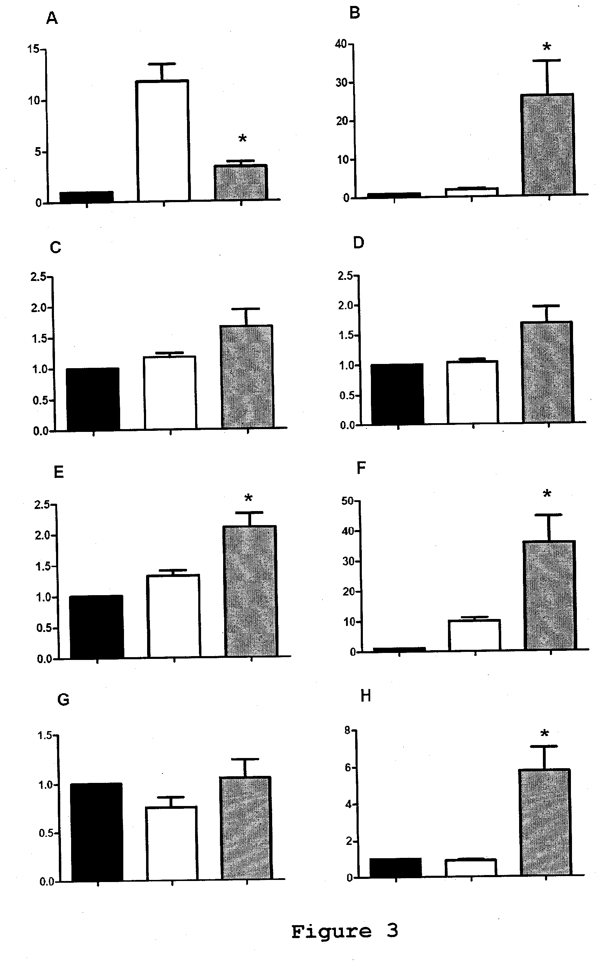

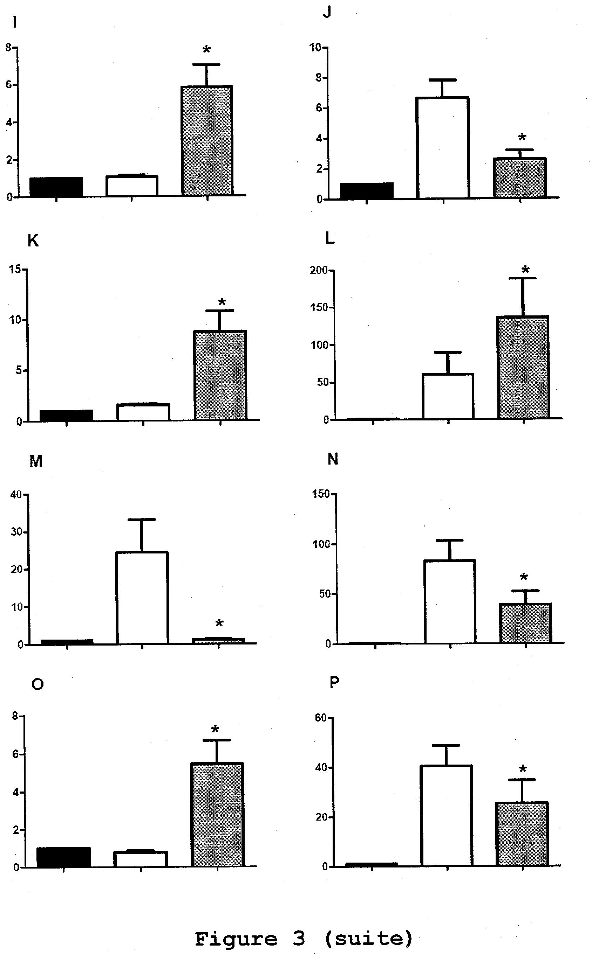

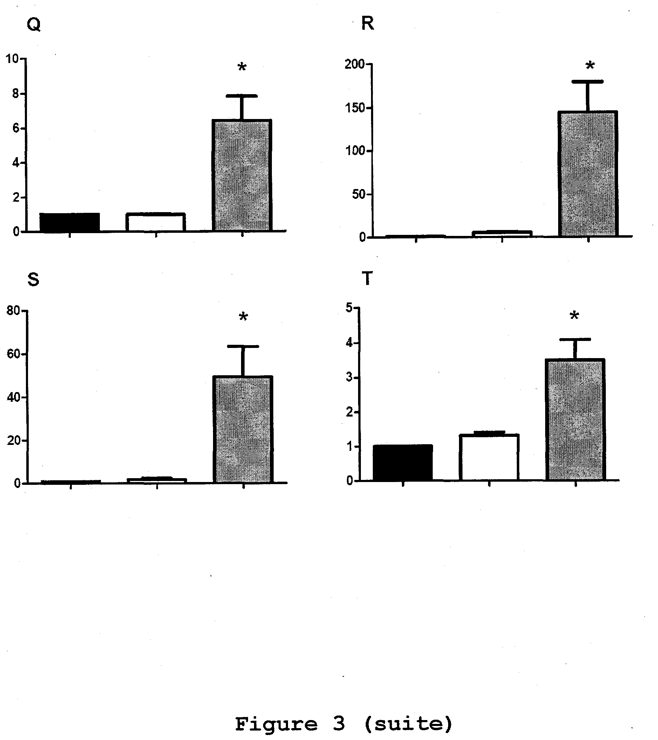

Figure 3 : La figure 3 montre l'expression des gènes de TNF alpha (A), IL-10 (B), HLA-DRA (C), CIITA (D), IRAK-M (E), ABIN-3 (F), LY64 (G), S100A9 (H), S100A8 (I), GBP1 (J), MMP7 (K), CXCL1 (L), CXCL10 (M), COX2 (N), FCN1 (O), TNFAIP6 (P), CXCL7 (Q), CXCL5 (R), PID1 (S) et RHOU (T) par des PBMCs, issues de 7 volontaires sains, stimulées par du LPS et quantifiées par qRT-PCR après 45 heures. L'axe des ordonnées représente les taux d'induction d'expression des gènes cités ci-dessus. Les histogrammes noirs représentent les contrôles négatifs (sans stimulation). Les histogrammes blancs correspondent aux cellules stimulées une seule fois avec 100 ng / mL de LPS et les histogrammes gris représentent les cellules soumises à deux stimulations par du LPS (2 ng / ml puis 100 ng/ ml). Les données sont représentées en moyenne +/- écart type. Un test appareillé de Wilcoxon a été réalisé pour l'analyse statistique des résultats: *, signifie que p<0,05 vs cellules stimulées une fois au LPS.

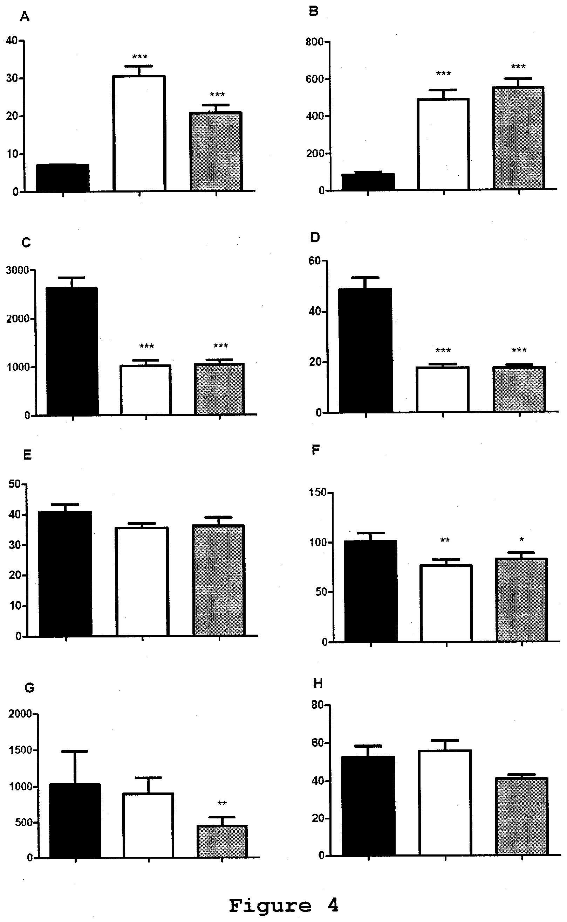

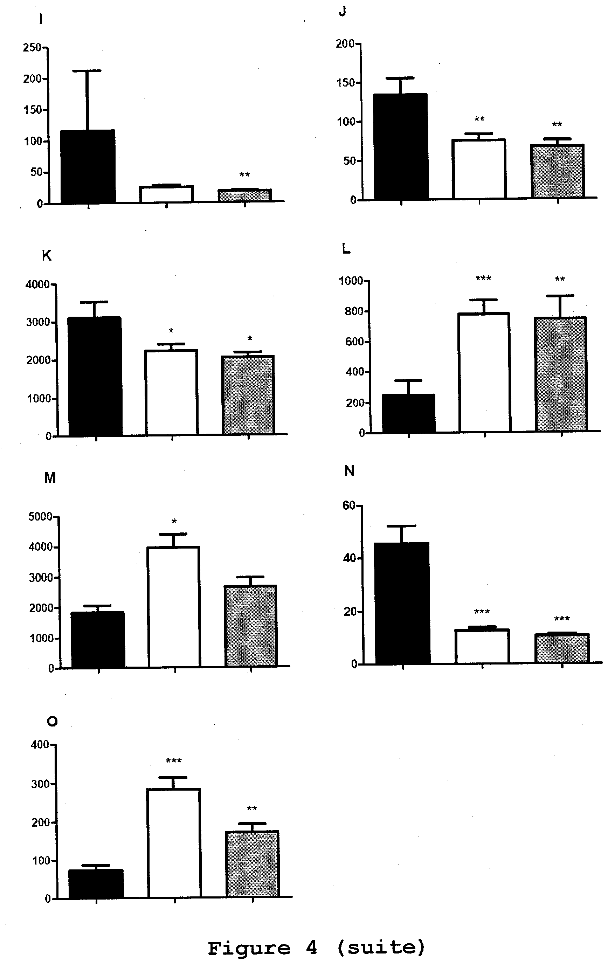

Figure 4 : La figure 4 représente l'expression des gènes de IL-10 (A), IRAK-M (B), HLA-DRA (C), CIITA (D), TNF-α (E),LY64 (F), GBP1 (G), CXCL1 (H), CXCL10 (I), COX2 (J), FCN1 (K), TNFAIP6 (L), CXCL7 (M), PID1 (N) et RHOU (O) quantifiées par biopuces à partir de sang total de 19 patients en choc septique, au diagnostic de choc (H0) et 48 heures après (H48) ou de sang de 9 volontaires sains. L'axe des ordonnées représente l'intensité de fluorescence de chacune des sondes d'hybridation : (A) 207433_at pour l'IL-10; (B) 213817_at pour IRAK-M (C) 208894_at pour HLA-DRA ; (D) 205101_at pour CIITA; (E) 207113_s_at pour TNF-α; (F) 206206_at pour LY64; (G) 202269_x_at pour GBP1 ; (H) 204470_at pour CXCL1 ; (I) 204533_at pour CXCL10 (J) ; 204748_at pour COX2 (K) ; 205237_at pour FCN1 (L) ; 206026_s_at pour TNFAIP6 (M) ; 214146_s_at pour CXCL7 (N) ; 219093_at pour PID1 et (O) 223168_at pour RHOU. Les histogrammes noirs représentent les volontaires sains, les histogrammes blancs correspondent aux patients à H0 et les histogrammes gris représentent les patients à H48. Les données sont représentées en moyenne +/- écart type. La comparaison entre les volontaires sains et les patients a été effectuée par un test de Mann Whitney (*, signifie que p<0,05 ; **, signifie que p<0,01 et ***, signifie que p<0,001).

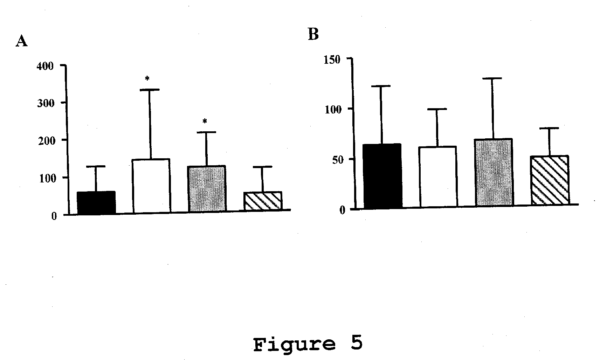

Figure 5: Les figures 5A et 5B représentent respectivement les concentrations de TNF-α et d'IL-10 produits par des PBMCs, issues de 10 volontaires sains, stimulées deux fois par du LPS (2 ng / ml puis 100 ng / ml) en présence ou non d'immunostimulants. Le dosage a été effectué par un test ELISA sur des surnageants de culture obtenus après 45 heures. L'axe des ordonnées représentent les concentrations protéiques (pg / ml) de TNF-α (figure 4A) et d'IL-10 (figure 4B). Les histogrammes noirs représentent les cellules stimulées deux fois par du LPS sans drogue (contrôle). Les histogrammes blancs correspondent aux cellules stimulées deux fois par du LPS en présence d'IFN-γ1b (Imukin™, Boehringer, Ingelheim, Austria) à 100 ng / ml, les histogrammes gris en présence de 100 ng / ml de GM-CSF (Sigma-Aldrich, Deisenhofen, Germany) et les histogrammes rayés en présence de 100 ng / ml de FLT3-L (Sigma-Aldrich, Deisenhofen, Germany). Les données sont représentées en moyenne +/- écart type. Un test appareillé de Wilcoxon a été réalisé pour l'analyse statistique des résultats en corrigeant par le nombre de test effectué : *, signifie que p<0,017 vs cellules stimulées deux fois au LPS sans drogue.

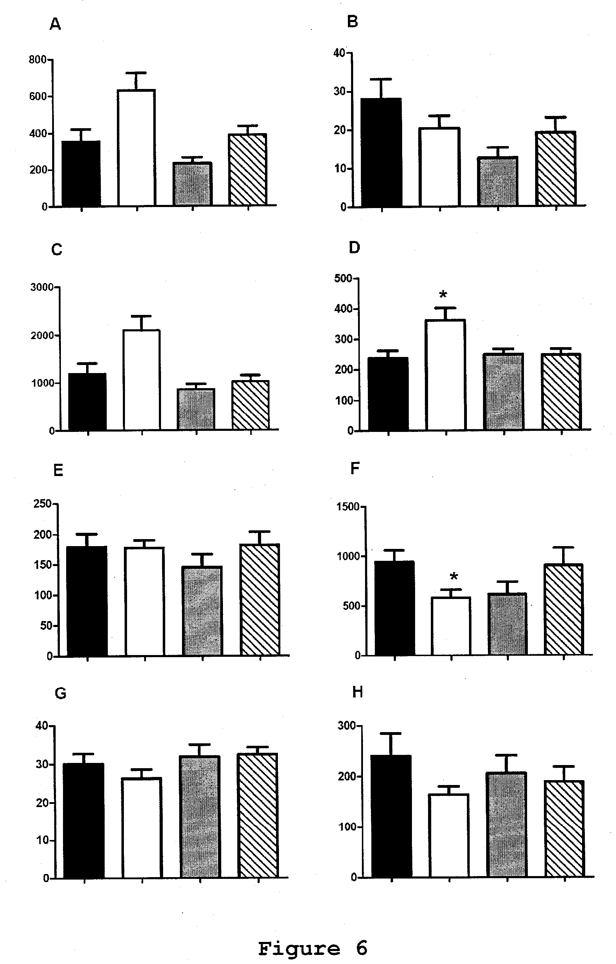

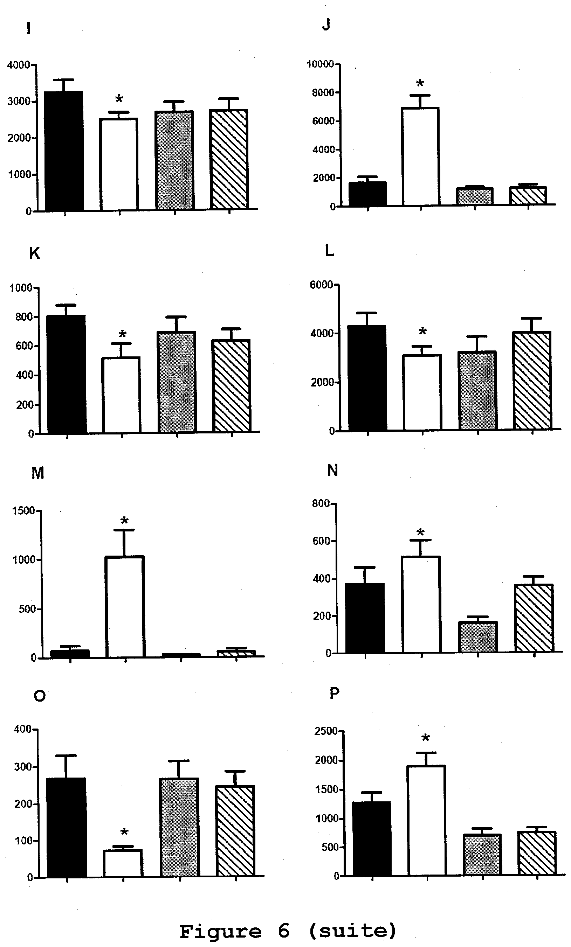

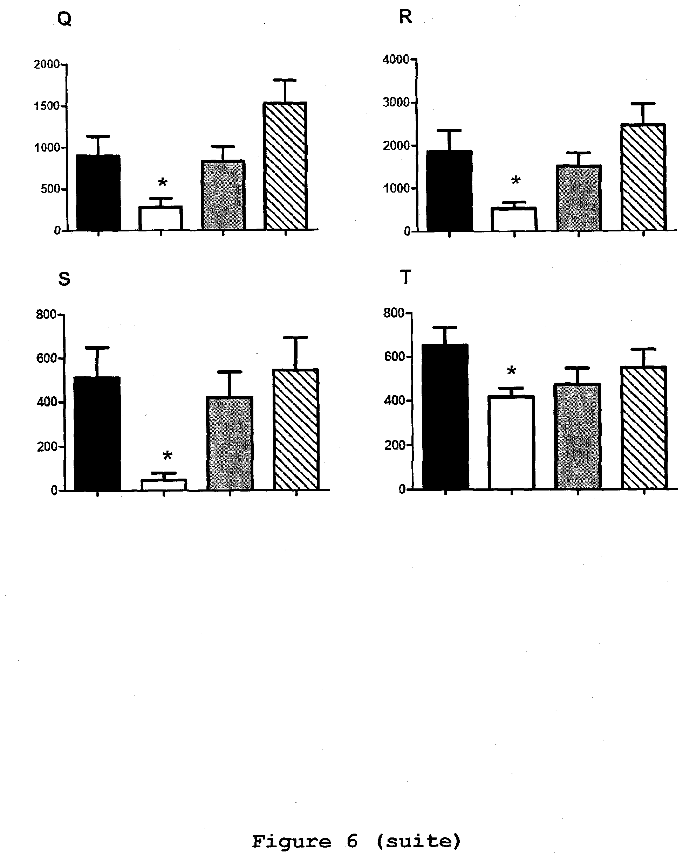

Figure 6: La figure 6 montre l'expression des gènes de TNF alpha (A), IL-10 (B), HLA-DRA (C), CIITA (D), IRAK-M (E), ABIN-3 (F), LY64 (G), S100A9 (H), S100A8 (I), GBP1 (J), MMP7 (K), CXCL1 (L), CXCL10 (M), COX2 (N), FCN1 (O), TNFAIP6 (P), CXCL7 (Q), CXCL5 (R), PID1 (S) et RHOU (T) par des PBMCs, issues de 7 volontaires sains, stimulées deux fois par du LPS (2 ng /ml puis 100 ng / ml) en présence ou non de drogues, et quantifiées par biopuces après 45 heures. L'axe des ordonnées représente l'intensité de fluorescence de chacune des sondes d'hybridation : (A) 207113_s_at pour le TNFa ; (B) 207433_at pour l'IL-10; (C) 208894_at pour HLA-DRA ; (D) 205101_at pour CIITA; (E) 213817_at pour IRAK-M ; (F) 220655_at pour ABIN-3 ; (G) 206206_at pour LY64; (H) 203535_at pour S100A9 ; (I) 202917_s_at pour S100A8 ;(J) 202269_x_at pour GBP1 ; (K) 204259_art pour MMP7 ; (L) 204470_at pour CXCL1 ; (M) 204533_at pour CXCL10 ; (N) 204748_at pour COX2; (O) 205237_at pour FCN1; (P) 206026_s_at pour TNFAIP6; (Q) 214146_s_at pour CXCL7; (R) 215101_s_at pour CXCL5 ; (S) 219093_at pour PID1 et (T) 223168_at pour RHOU. Les histogrammes noirs représentent les cellules stimulées deux fois par du LPS sans drogue (contrôle). Les histogrammes blancs correspondent aux cellules stimulées deux fois par du LPS en présence d'IFN-γ1b à 100 ng / ml, les histogrammes gris en présence de 100 ng / ml de GM-CSF et les histogrammes rayés en présence de 100 ng / ml de FLT3-L. Les données sont représentées en moyenne +/- écart type. Un t-test appareillé a été réalisé pour l'analyse statistique des résultats en corrigeant par le nombre de test effectué : *, signifie que p<0,05 vs cellules stimulées deux fois au LPS sans immunostimulant.

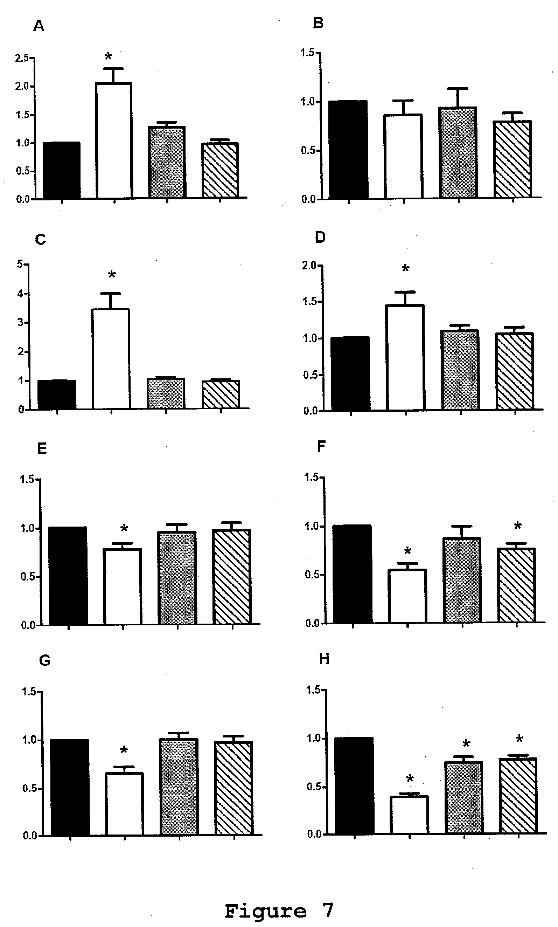

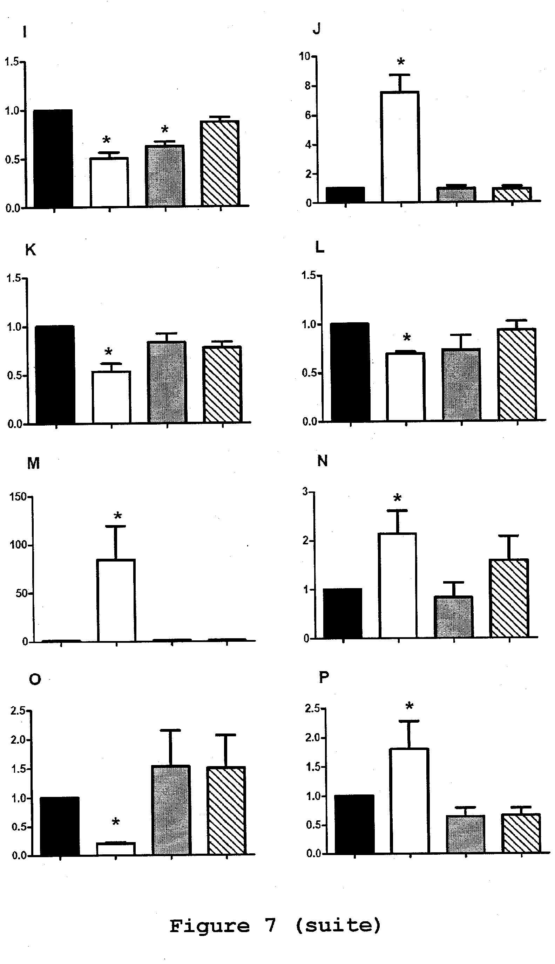

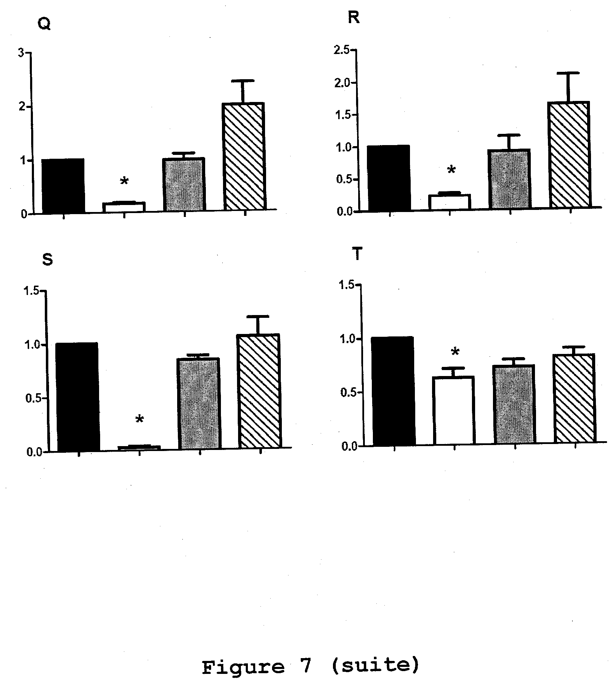

Figure 7 : La figure 7 montre l'expression des gènes de TNFa (A), IL-10 (B), HLA-DRA (C), CIITA (D), IRAK-M (E), ABIN-3 (F), LY64 (G), S100A9 (H) , S100A8 (I), GBP1 (J), MMP7 (K), CXCL1 (L), CXCL10 (M), COX2 (N), FCN1 (O), TNFAIP6 (P), CXCL7 (Q), CXCL5 (R), PID1 (S) et RHOU (T) par des PBMCs, issues de 7 volontaires sains, stimulées deux fois par du LPS (2 ng /ml puis 100 ng / ml) en présence ou non de drogues, et quantifiées par qRT-PCR après 45 heures. L'axe des ordonnées représente les taux d'induction d'expression des gènes cités ci-dessus. Les histogrammes noirs représentent les cellules stimulées deux fois par du LPS sans drogue (contrôle). Les histogrammes blancs correspondent aux cellules stimulées deux fois par du LPS en présence d'IFN-γ1b à 100 ng / ml, les histogrammes gris en présence de 100 ng / ml de GM-CSF et les histogrammes rayés en présence de 100 ng / ml de FLT3-L. Les données sont représentées en moyenne +/- écart type. Un test appareillé de Wilcoxon a été réalisé pour l'analyse statistique des résultats en corrigeant par le nombre de test effectué : *, signifie que p<0,05 vs cellules stimulées deux fois au LPS sans immunostimulant.

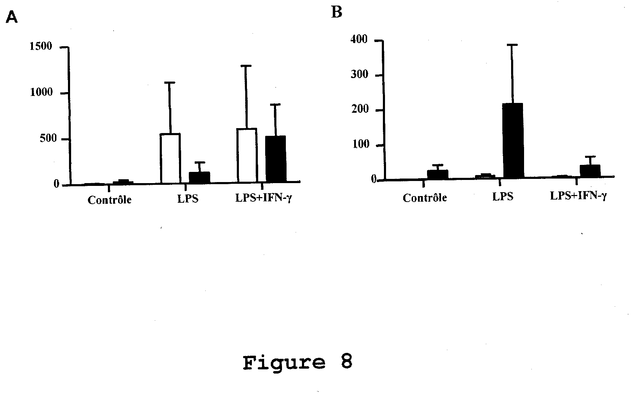

Figure 8 : les figures 8A et 8B représentent respectivement les concentrations de TNF-α et d'IL-10 produites par des cellules du sang total, issues de 5 patients en choc septique prélevés entre J1 et J3 et 5 volontaires sains, stimulées ou non (contrôle) par du LPS à 100 ng / ml +/- 100 ng / ml d'IFN-γ1b. Les dosages protéiques ont été réalisés par un test ELISA sur des surnageants de culture obtenus après 15 heures. L'axe des ordonnées représentent les concentrations protéiques (pg / ml) de TNF-α (figure 6 A) et d'IL-10 (figure 6 B). Les histogrammes noirs représentent les patients en choc septique et les histogrammes en blanc représentent les volontaires sains. Les données sont représentées en moyenne +/- écart type.

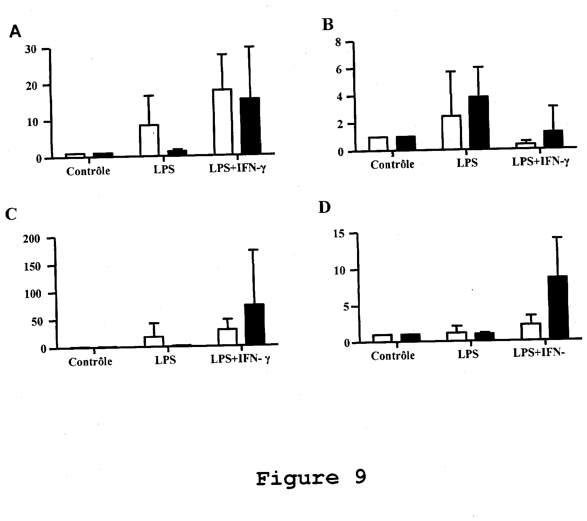

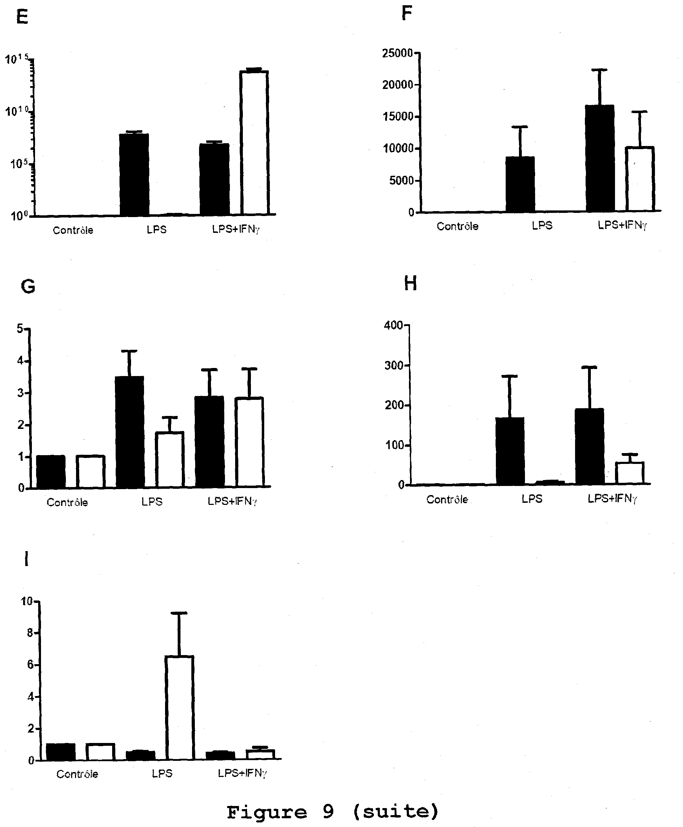

Figure 9 : La figure 9 représente l'expression des gènes de TNF-α (A), IL-10 (B), HLA-DRA (C), CIITA (D), GBP1 (E), CXCL10 (F), COX2 (G), TNFAIP6 (H) et CXCL5 (I) par des cellules du sang total, issues de 5 patients en choc septique prélevés entre J1 et J3 et 5 volontaires sains, stimulées ou non (contrôles) par du LPS à 100 ng / ml +/- 100 ng / ml d'IFN-γ1b. La quantification de ces gènes a été effectuée par qRT-PCR sur les culots cellulaires obtenus après 15 heures. L'axe des ordonnées représente les taux d'induction d'expression des gènes cités ci-dessus. Les histogrammes noirs représentent les patients en choc septique et les histogrammes en blanc représentent les volontaires sains.

### EXEMPLES

Les exemples suivants sont donnés à titre illustratif et n'ont aucun caractère limitatif. Ils permettent de mieux comprendre l'invention.

### Matériels et méthodes

### Préparation des PBMCs et paramètres expérimentaux du modèle de tolérance à l'endotoxine (ET)

Les PBMCs issues de différents volontaires sains ont été isolées à partir de sang prélevé en poches traitées au citrate par gradient de Ficoll (Amersham Biosciences, Björkgatan, Suède), puis lavées avec du PBS tandis que les hématies restantes ont été lysées. Les cellules ont été mises en culture dans des plaques 24 puits à raison de 2.10⁶ cellules / ml dans du milieu X-Vivo 20 (Lonza, Verviers, Belgique). Afin d'induire une première exposition au LPS, les PBMCs ont été mises en culture en présence ou en absence (groupe contrôle) de 2 ng / ml d'un mélange de LPS issu d*'Escherichia coli* O55:B5, O127:B8 and O111:B4 (Sigma-Aldrich, Deisenhofen, Allemagne) et incubées une nuit (15 heures) à 37 °C et 5 CO₂. Après lavage, les cellules ont été incubées pendant 24 autres heures en présence ou en absence (groupe contrôle stimulé ou non avec du LPS) d'IFN-γ1b (Imukin TM, Boehringer, Ingelheim, Autriche), de GM-CSF ou de Flt3-L (Sigma-Aldrich). Enfin, les cellules ont été restimulées par une seconde dose de LPS à 100 ng / ml pendant 6 heures. Les protéines TNF-α et IL-10 ont été dosées dans ces surnageants tandis que l'expression ARNm a été quantifiée à partir des culots cellulaires. Pour chaque condition, les surnageants et les culots cellulaires ont été récupérés et conservés à -80°C pour le dosage ELISA des protéines TNF-α et IL-10 et à -20°C pour l'extraction ARN et la quantification par qRT-PCR.

### Test fonctionnel par ELISA

La détection des concentrations de TNF-α et d'IL-10 dans les surnageants de culture de PBMCs a été réalisée via les kits ELISA commercialisés par la société R&D Sytem (Mineapolis, USA).

### Extraction d'ARN

Les ARN totaux ont été extraits à partir des PBMCs à l'aide du kit RNeasy Plus Mini TM (Qiagen, Hilden, Allemagne) ou à partir de sang total en utilisant le kit QIAamp RNA Blood Mini TM (Qiagen), selon les instructions du fabriquant. Pour chaque extraction d'ARN, l'ADN résiduel génomique a été digéré sur les colonnes gDNA Eliminator (Qiagen) TM, puis les ARN ont été élués dans 30 µl d'un tampon d'élution. Les concentrations d'ARN ont été mesurées pour chaque échantillon à l'aide du Qubit TM (Invitrogen, Carlsbad, CA, USA), conformément aux instructions du fabriquant.

### Analyse par Biopuce

L'analyse d'expression des gènes a été faite à partir de 50ng d'ARN amplifié avec le kit WT-Ovation RNA Amplification System de Nugen (NuGEN Technologies, Inc. , San Carlos, CA, USA). L'ARN amplifié a été hybridé sur des puces Affymetrix® HG-U133Plus 2.0 suivant les instructions du fabricant (Affymetrix®, Santa Clara, CA, USA). L'analyse des données Affymetrix débute par la capture de l'image de la biopuce par le scanner GeneChip® 3000 d'Affymetrix. Les données ont été ensuite normalisées par RMA (Robust Multiple - Array Average) (Irizarry RA, Biostatistics 2003). Toutes les données basées sur l'intensité du signal ont été utilisées après la normalisation.

### Synthèse des ADNc par transcription inverse (RT) et analyse par PCR quantitative (qPCR)

L'expression des ARN messagers (ARNm) a été quantifiée à l'aide de la qPCR. Brièvement, les ADNc ont été synthétisés à partir de 100 ng d'ARN total grâce à une étape de transcription inverse en utilisant la SuperScript® VILO™ system (Invitrogen).Les réactions PCR ont ensuite été réalisées sur l'appareil LightCycler® 480 utilisant soit le marqueur fluorescent, SYBR Green I Master Mix, selon les instructions du fabricant (Roche Molecular Biochemicals, Indianapolis, IN, USA), soit le Probes master mix, selon les instructions du fabricant (Roche Molecular Biochemicals, Indianapolis, IN, USA) . Pour l'amplification, le volume réactionnel était de 20 µl, et les cycles comprenaient une étape de dénaturation initiale à 95 °C pendant 5 minutes (1 cycle), suivi par 45 cycles d'amplification (20 secondes à 95 °C, 15 secondes hybridation à 68-58 °C et 15 secondes d'extension à 72 °C), une courbe de fusion à 95 °C 1 seconde, 60 °C 10 secondes et 95 °C pendant 5 minutes, et pour finir un refroidissement à 40 °C pendant 30 secondes. Vingt gènes ont été quantifiés dans cette étude: TNF-α, l'IL-10, HLA-DRA, CIITA, CX3CR1, IRAK-M, ABIN-3, LY64, S100A9, S100A8, GBP1, MMP7, CXCL1, CXCL10, COX2, FCN1, TNFAIP6, CXCL7, CXCL5, PID1 et RHOU. Les amorces et/ou sondes utilisées ont été soit optimisées par la société Search-LC (Search-LC, Heidelberg, Allemagne), pour le gène de ménage PPIB, codant pour la cyclophiline B et le TNF-α, soit par la société Applied Biosystems (Life Technologies Corporation, Carlsbad, California, USA) pour les gènes GBP1 (référence Hs00977005_ml), MMP7 (réf. : Hs01042796_m1), CXCL1 (réf. : Hs00236937_m1), CXCL10 réf. : Hs00171042_m1), COX2 (réf. : Hs00153133_m1), FCN1 (réf. : Hs00157572_m1), TNFAIP6 (réf. : Hs01113602_m1, CXCL7 (réf. : Hs00234077_m1), CXCL5 (réf. : Hs00171085_m1), PID1 (réf. : Hs00952182_m1) et RHOU (réf. : Hs00221873_m1) et pour le gène de ménage PPIB (réf. :Hs00165719_m1), soit en interne, pour les autres gènes étudiés (IL-10, HLA-DRA, CIITA, CD180 (LY64), IRAK-M, ABIN-3, S100A9 et S100A8) (voir tableau ci-dessous). Plusieurs dilutions d'ADNc ont été préparées en quadruplicate pour générer les gammes standards. Les résultats ont été normalisés par rapport au gène de référence (*PPIB*), ainsi qu'à un calibrateur interne afin de minimiser l'impact du manipulateur et l'efficacité des différentes enzymes. Les résultats ont été exprimés en « fold change » de ratio normalisés et n'étaient inclus dans l'analyse que lorsque les valeurs ARNm étaient comprises dans la gamme standard de chacun des gènes étudiés.

**Tableau**

| **Gene** | **N° accession GenBank** | **Amorces** | | **SEQ ID NOs** |
|---|---|---|---|---|
| *IL-10* | NM_000572.2 | 5'-AATAAGGTTTCTCAAGGGGCT-3' | sens | 1 |
| | | 5'-AGAACCAAGACCCAGACATCAA-3' | anti-sens | 2 |
| *HLA-DRA* | NM_019111.4 | 5'-GCCAACCTGGAAATCATGACA-3' | sens | 3 |
| | | 5'-AGGGCTGTTCGTGAGCACA-3' | anti-sens | 4 |
| *CIITA* | NM_000246.3 | 5'-GCTGGGATTCCTACACAATGC-3' | sens | 5 |
| | | 5'-CGGGTTCTGAGTAGAGCTCAATCT-3' | anti-sens | 6 |
| *IRAK-M* | NM_007199.2 | 5'-TTTGAATGCAGCCAGTCTGA-3' | sens | 7 |
| | NM_001142523.1 | 5'-GCATTGCTTATGGAGCCAAT-3' | anti-sens | 8 |
| *ABIN-3* | NM_024873.4 | 5'-GAATTCCCAGATAAAAGCTTGT-3' | sens | 9 |
| | NM 001128843.1 | 5'-GACAGTCTGGTGGGTGCTC-3' | anti-sens | 10 |
| *S100A9* | NM_002965.3 | 5'-TCAAAGAGCTGGTGCGAAAA-3' | sens | 11 |
| | | 5'-AACTCCTCGAAGCTCAGCTG-3' | anti-sens | 12 |
| *S100AB* | NM_002964.4 | 5'-ATTTCCATGCCGTCTACAGG-3' | sens | 13 |
| | | 5'-CACCAGAATGAGGAACTCCT-3' | anti-sens | 14 |
| *LY64* | NM_005582.2 | 5'-GCATTGAGAAAGAAGCCAACAA-3' | sens | 15 |
| | | 5'-GAAAAGTGTCTTCATGTATCC-3' | anti-sens | 16 |

### Approche transcriptionnelle sur biopuces chez des patients en choc septique

Plusieurs échantillons de sang collectés directement dans des tubes PAXgeneTM (PreAnalytiX a Qiagen / BD Company) ont été obtenus à partir de 19 patients en choc septique au moment du diagnostic de choc (i.e. dans les 2 heures suivant le début du traitement vasopresseur) et 48 heures après, comme décrit précédemment [9]. L'ARN total a été extrait à l'aide du kit PAXgene Blood RNA kit™ (PreAnalytix). Une étape de digestion à la DNAse (Qiagen) a été effectuée au cours de l'extraction afin d'éliminer toute trace d'ADN. L'intégrité et la qualité des ARN ont été vérifiées par électrophorèse capillaire sur le bioanalyseur 2100 Agilent™ (Agilent). Les biopuces ont été réalisées suivant les instructions du fabriquant (Affymetrix®). La réaction de transcription inverse des ARNm a été réalisée avec le protocole d'Affymetrix à partir de 3µg de l'ARN total. Une amorce oligonucléotidique polyT a été utilisée pour cibler uniquement les ARNm contenus dans la solution des ARN totaux pendant la transcription inverse. L'ensemble des étapes a été réalisé avec le kit Afffymetrix - GeneChip® One-Cycle Target Labeling and Control Reagents. Les produits de la transcription inverse (ADNc) ont ensuite été utilisés pour la synthèse de l'ARNc marqué par la biotine pendant 16 heures à 37°C. Après l'étape de purification de l'ARNc pour enlever les dNTP non incorporés, les ARNc ont été quantifiés et fragmentés. Le contrôle de la fragmentation se fait par électrophorèse capillaire (Agilent). L'hybridation de l'ARNc est réalisée avec la biopuce GeneChipHuman Genome U133 Plus 2 avant l'étape d'amplification du signal par l'incubation avec le mélange SAPE contenant la Streptavidine Phycoerythrine puis les anticorps IgG de chèvre anti-streptavidine mélangés avec l'anticorps biotinylé anti-IgG de chèvre. Cette dernière étape utilise la plateforme Fluidic FS450. L'analyse des données Affymetrix débute par la capture de l'image de la biopuce par le scanner GeneChip® 3000 d'Affymetrix. Les données ont été ensuite normalisées par RMA (Robust Multiple -Array Average) (Irizarry RA, Biostatistics 2003). Toutes les données basées sur l'intensité du signal ont été utilisées après la normalisation.

### Effet de l'IFN-γ dans un modèle de sang total de patients en choc septique

Le sang de 5 patients en choc septique a été collecté en tube EDTA, entre J1 et J3 suivant le début du choc, ainsi que chez 5 volontaires sains. Après centrifugation et retrait du plasma, 3 ml de sang ont été dilué au ½ dans 3 ml de milieu RPMI 1640 (Eurobio, Courtaboeuf, France) puis mis en culture en présence ou en absence (groupe contrôle) de 100 ng / ml de LPS + / - 100 ng / ml d'IFN-γ1b (Imukin) pendant une nuit à 37 °C et 5 % CO₂ (15 heures). Pour chaque condition, les surnageants ainsi que les culots cellulaires ont été récupérés et conservés à -80°C pour le dosage ELISA du TNF-α et de l'IL-10 et à -20°C pour l'extraction ARN et la quantification par qRT-PCR, comme décrit précédemment.

### Analyses statistiques

Les résultats sont exprimés en moyenne ± écart type (SD). L'analyse statistique a été réalisée par le test appareillé de Wilcoxon.

### Résultats

### Description du modèle d'ET

Les modèles d'ET sont caractérisés par une réduction de la production de TNF-α associée à une augmentation de la sécrétion d'IL-10 à la suite d'une seconde stimulation par du LPS. La production de ces cytokines a donc été mesurée dans le surnageant de culture de PBMCs suite à une ou deux stimulations par du LPS. Comme montré dans la figure 1A, les cellules stimulées deux fois par du LPS produisent de faibles quantités de TNF-α (58,5 ± 68,3 pg /mL) comparées aux cellules stimulées uniquement une fois (1208 ± 594 pg /mL). En revanche, comme montré à la figure 1B, ces mêmes cellules sécrètent de plus fortes concentrations d'IL-10 (63,2 ± 58,2 pg /mL) en comparaison aux cellules stimulées une fois (8,4 ± 6,9 pg /mL). En réponse à une seconde stimulation endotoxinique, les cellules déjà stimulées relarguent moins de TNF-α (58,5 ± 68,3 pg /mL) que les cellules stimulées une seule fois (1208 ± 594 pg /mL) et produisent plus d'IL-10 (63,2 ± 58,2 vs 8,4 ± 6,9 pg /mL) que les cellules stimulées une fois). Ces résultats sont confirmés au niveau ARNm puisqu'une diminution significative de l'expression du TNF-α (figure 2A) couplée à une augmentation de l'expression génique de l'IL-10 (figure 2B) dans les cellules stimulées deux fois par du LPS ont été observées en comparaison au PBMCs stimulées une seule fois.
Une analyse de l'expression génique de PBMCs soumises à une ou deux stimulations au LPS a été faite sur des biopuces Affymetrix. Les gènes dont l'expression diffère le plus entre une ou deux stimulation au LPS (p<0.05 et différence d'expression >2) ainsi que des gènes connus dans la littérature pour être impliqués dans cet état réfractaire de l'ET ou la phase d'immunosuppression induite par les états septiques sévères sont représentés à la Figure 2. La seconde stimulation par l'endotoxine bactérienne est accompagnée par une augmentation de l'expression d'ARNm de IL-10 (B),HLA-DRA (C), CIITA (D), IRAK-M (E), ABIN-3 (F) tandis que l'expression de TNF alpha (A) et de LY64 (G) est diminuée. L'expression génique des alarmines S100A9 (H) et S100A8 (I), des chemokines CXCL1 (L), CXCL5 (R), et CXCL7 (Q), de MMP7 (K), FCN1 (O), PID1 (S) et RHOU (T) augmentent également suite à une deuxième stimulation au LPS. Enfin, l'expression des gènes induits par l'interféron GBP1 (J) et CXCL10 (M) ainsi que de TNFAIP6 (P) diminue.
Ces données ont ensuite été confirmées par qRT-PCR (Figure 3) où le même profil que celui obtenu sur les biopuces a été observé. En réponse à un second challenge endotoxinique, l'incapacité à produire et exprimer le TNF-α combinée à l'augmentation de la production et de l'expression d'IL-10, des cellules ayant été préalablement exposées à une première dose de LPS, indique clairement le développement d'un état d'ET.

### Analyse de l'expression des gènes chez des patients en choc septique

Afin d'illustrer la pertinence clinique du modèle, l'expression de ces gènes chez des patients en choc septique a été étudiée rétrospectivement, au moment du diagnostic du choc et 48 heures plus tard. L'expression des gènes a été évaluée par une approche sur biopuces dans une cohorte de 19 patients décrite précédemment [9] et 9 volontaires sains. Comme observé dans le modèle d'ET, les résultats montrent une augmentation de l'expression de l'IL-10, IRAK-M, CXCL7 et RHOU (figure 4A, B, M et O) couplée à une diminution de l'expression de Ly64, GBP1 et CXCL10 (figure 4F, G et I) chez les patients en choc septique au moment du diagnostic et même 48 heures plus tard comparé aux volontaires sains. En parallèle, une légère diminution de l'expression du TNF-α est observée chez les patients (figure 4 E).

Dans l'ensemble, les données cliniques confirment les modifications ARNm observées *ex vivo* dans le modèle d'ET et soulignent la pertinence clinique de ce modèle.

### L'IFN-γ et le GM-CSF améliorent significativement la production de TNF-α dans les PBMCs désactivées par LPS

Comme indiqué dans la figure 1, les PBMCs stimulées une première fois au LPS présentent une diminution marquée de leur capacité de production en TNF-α en réponse à une seconde exposition à l'endotoxine. Par la suite, il a été investigué, dans 7 expériences successives, si une incubation de 24 heures en présence de différentes drogues immunomodulatrices (IFN-γ, GM-CSF et Flt3-L) pouvait restaurer cette synthèse en TNF-α et diminuer celle d'IL-10. L'IFN-γ et le GM-CSF atteignent cet objectif en induisant une augmentation de la production en TNF-α (figure 5 A). Il est intéressant de noter que les drogues testées n'ont d'impact que sur le renforcement du versant pro-inflammatoire, mais restent sans effet sur la diminution de production de l'IL-10 (figure 5B).

### Monitoring ARNm de l'effet des drogues immunostimulantes au cours de l'ET

Par la suite, dans ces mêmes expériences montrant un effet bénéfique de l'IFN-γ et du GM-CSF sur la restauration de la synthèse de TNF-α en réponse à une seconde stimulation au LPS, la modulation d'expression d'un panel de gènes a été étudiée par biopuces (Figure 6) puis validée par qRT-PCR (Figure 7).
On observe une induction significative de l'expression ARNm du TNF-α en présence d'IFN-γ comparé aux valeurs contrôles (figure 6A). L'addition des drogues induit une légère diminution de l'expression ARNm de l'IL10 (figure 6B). En parallèle, une augmentation significative de l'expression génique de HLA-DRA et CIITA est observée lorsque les cellules prétraitées au LPS sont incubées avec de l'IFN-γ (figure 6C, 6D). Concernant les régulateurs négatifs de la voie de signalisation du LPS, l'expression d'ABIN-3 diminue significativement en présence d'IFN-γ (figure 6E, 6F). L'expression des gènes LY64 et IRAKM diminuent également en présence d'IFN-γ (figure 5G). Les trois drogues diminuent l'expression ARNm des alarmines S100A9 et S100A8 (figure 6H, 6I). De plus, la présence d'IFN-γ augmente significativement l'expression des gènes GBP1 (J), CXCL10 (M), COX 2 (N), TNFAIP6 (P) et diminue significativement celle de MMP7 (K), CXCL1 (L), FCN1 (O), CXCL7 (Q), CXCL5 (R), PID1 (S) et RHOU (T).

### Effet de l'IFN-γ sur le sang total de patients en choc septique

Finalement, les résultats obtenus ont été confirmés à partir de sang total de patients en choc septique. Comme attendu, en réponse à une stimulation de LPS, les cellules de patients produisent moins de TNF-α (figure 8 A) et plus d'IL-10 (figures 8 B) que celles des volontaires sains, soulignant le développement d'un état d'ET. L'addition d'IFN-γ restaure totalement la sécrétion de TNF-α et inhibe complètement la production en IL-10 chez les patients en choc septique. Ces résultats protéiques sont confirmés au niveau ARNm (figure 9A, B). De plus, l'expression de GBP1 (Figure 9 E), de CXCL10 (Figure 9F), de COX2 (Figure 9G) et de TNFAIP6 (Figure 9H) est très fortement réduite dans les cellules de patients suite à une stimulation au LPS par rapport à celle des volontaires sains. L'addition d'IFN-γ restaure l'expression de ces gènes. Parallèlement, les cellules de patients expriment beaucoup plus de CXCL5 (Figure 9I) en réponse à une stimulation au LPS et l'expression de CXCL5 redevient équivalente à celle observée chez les donneurs sains après addition d'IFN-γ. Enfin, l'expression génique de HLA-DRA et de CIITA augmentent également fortement chez les patients en présence d'IFN-γ (figure 9C, D).

### Références bibliographiques

1. Kricka et al., Clinical Chemistry, 1999, n° 45(4), p.453-458.
2. Keller G.H. et al., DNA Probes, 2nd Ed., Stockton Press, 1993, sections 5 et 6, p.173-249.
3. Bustin SA Journal of molecular endocrinology, 2002, 29 : 23-39.
4. Giulietti A Methods, 2001, 25 : 386-401.
5. Nisonoff A. et al., 1960, Science, 132 : 1770-1771).
6. Skerra A., 1993, Curr. Opin. Immunol., 5 : 256-262.
7. Huston P. et al., 1988, Proc. Natl.Acad. Sci.USA, 85 : 5879-5883.
8. Munoz C, J Clin Invest 1991.
9. Venet F, Shock 2010.

### SEQUENCE LISTING

STRATICHOC PCT.ST25
<110> bioMérieux Hospices Civils de Lyon
<120> Procédé et kit pour déterminer in vitro le statut immunitaire d'un individu
<130> STRATICHOC
<150> FR 1150646
   <151> 2011-01-27
<160> 16
<170> PatentIn version 3.3
<210> 1
   <211> 21
   <212> DNA
   <213> Artificial
<220>
   <223> Amorce
<400> 1
   aataaggttt ctcaaggggc t 21
<210> 2
   <211> 22
   <212> DNA
   <213> Artificial
<220>
   <223> Amorce
<400> 2
   agaaccaaga cccagacatc aa 22
<210> 3
   <211> 21
   <212> DNA
   <213> Artificial
<220>
   <223> Amorce
<400> 3
   gccaacctgg aaatcatgac a 21
<210> 4
   <211> 19
   <212> DNA
   <213> Artificial
<220>
   <223> Amorce
<400> 4
   agggctgttc gtgagcaca 19
<210> 5
   <211> 21
   <212> DNA
   <213> Artificial
<220>
   <223> Amorce
<400> 5
   gctgggattc ctacacaatg c 21
<210> 6
   <211> 24
   <212> DNA
   <213> Artificial
<220>
   <223> Amorce
<400> 6
   cgggttctga gtagagctca atct 24
<210> 7
   <211> 20
   <212> DNA
   <213> Artificial
<220>
   <223> Amorce
<400> 7
   tttgaatgca gccagtctga 20
<210> 8
   <211> 20
   <212> DNA
   <213> Artificial
<220>
   <223> Amorce
<400> 8
   gcattgctta tggagccaat 20
<210> 9
   <211> 22
   <212> DNA
   <213> Artificial
<220>
   <223> Amorce
<400> 9
   gaattcccag ataaaagctt gt 22
<210> 10
   <211> 19
   <212> DNA
   <213> Artificial
<220>
   <223> Amorce
<400> 10
   gacagtctgg tgggtgctc 19
<210> 11
   <211> 20
   <212> DNA
   <213> Artificial
<220>
   <223> Amorce
<400> 11
   tcaaagagct ggtgcgaaaa 20
<210> 12
   <211> 20
   <212> DNA
   <213> Artificial
<220>
   <223> Amorce
<400> 12
   aactcctcga agctcagctg 20
<210> 13
   <211> 20
   <212> DNA
   <213> Artificial
<220>
   <223> Amorce
<400> 13
   atttccatgc cgtctacagg 20
<210> 14
   <211> 20
   <212> DNA
   <213> Artificial
<220>
   <223> Amorce
<400> 14
   caccagaatg aggaactcct 20
<210> 15
   <211> 22
   <212> DNA
   <213> Artificial
<220>
   <223> Amorce
<400> 15
   gcattgagaa agaagccaac aa 22
<210> 16
   <211> 21
   <212> DNA
   <213> Artificial
<220>
   <223> Amorce
<400> 16
   gaaaagtgtc ttcatgtatc c 21

## Revendications

1. Procédé pour déterminer *in vitro* le statut immunitaire global d'un individu septique pour la mise en place d'une thérapeutique la mieux ciblée en fonction de la réponse immunitaire établie selon lequel:
a. on dispose d'un échantillon sanguin de l'individu,
b. on dispose d'au moins deux réactifs spécifiques d'au moins deux produits d'expression d'au moins deux gènes cibles respectivement choisis parmi les gènes cibles suivants :
HLA-DRA, S100A9, S100A8, IRAK-M, LY64, CIITA, TNF-alpha, IL-10, GBP1, MMP7, CXCL1, CXCL10, COX2, FCN1, TNFAIP6, CXCL7, CXCL5, PID1 et RHOU,
c. on détermine l'expression desdits au moins deux gènes cibles, et
d. on compare l'expression desdits au moins deux gènes cibles respectivement avec une expression de référence, une modification dans l'expression desdits au moins deux gènes cibles par rapport à leur expression de référence étant un indicateur d'une modification du statut immunitaire de l'individu et une corrélation entre l'expression desdits au moins deux gènes cibles avec leur expression de référence étant un indicateur d'une normalité du statut immunitaire de l'individu.

2. Procédé selon la revendication 1, dans lequel l'échantillon sanguin est un échantillon est un échantillon de sang total.

3. Procédé selon la revendication 1 ou 2, dans lequel dans l'étape a. on extrait les cellules mononucléees du sang périphérique de l'échantillon sanguin et on réalise les étapes subséquentes b. à d. sur lesdites cellules mononucléees du sang périphérique.

4. Procédé selon l'une quelconque des revendications 1 à 3, dans lequel les réactifs spécifiques des produits d'expression des gènes cibles comprennent au moins une amorce d'amplification spécifique desdits gènes cibles.

5. Procédé selon l'une quelconque des revendications 1 à 4, dans lequel les réactifs spécifiques des produits d'expression des gènes cibles comprennent au moins une sonde d'hybridation spécifique desdits gènes cibles.

6. Procédé selon l'une quelconque des revendications 1 à 5, dans lequel les réactifs spécifiques des produits d'expression des gènes cibles comprennent au moins un anticorps.

7. Utilisation d'au moins deux réactifs spécifiques d'au moins deux produits d'expression d'au moins deux gènes cibles respectivement choisis parmi les gènes cibles suivants :
HLA-DRA, S100A9, S100A8, IRAK-M, LY64, CIITA, TNF-alpha, IL-10, GBP1, MMP7, CXCL1, CXCL10, COX2, FCN1, TNFAIP6, CXCL7, CXCL5, PID1 et RHOU, pour déterminer *in vitro* le statut immunitaire global d'un individu septique pour la mise en place d'une thérapeutique la mieux ciblée en fonction de la réponse immunitaire établie.

8. Utilisation selon le revendication 7, dans laquelle les réactifs spécifiques des produits d'expression desdits gènes cibles comprennent au moins une amorce d'amplification.

9. Utilisation selon l'une quelconque des revendications 7 ou 8, dans laquelle les réactifs spécifiques des produits d'expression desdits gènes cibles comprennent au moins une sonde d'hybridation.

10. Utilisation selon l'une quelconque des revendications 7 ou 8, dans laquelle les réactifs spécifiques des produits d'expression desdits gènes cibles comprennent au moins un anticorps.

## Patentansprüche

1. Verfahren zur Bestimmung des Gesamt-Immunstatus eines septischen Individuums *in vitro,* um eine Therapie umzusetzen, die je nach der festgestellten Immunantwort am stärksten zielgerichtet ist, bei dem man:
a. über eine Blutprobe des Individuums verfügt,
b. über mindestens zwei Reagenzien verfügt, die für mindestens zwei Expressionsprodukte von mindestens zwei Zielgenen spezifisch sind, die jeweils aus den folgenden Zielgenen ausgewählt sind:
HLA-DRA, S100A9, S100A8, IRAK-M, LY64, CIITA, TNF-alpha, IL-10, GBP1, MMP7, CXCL1, CXCL10, COX2, FCN1, TNFAIP6, CXCL7, CXCL5, PID1 und RHOU,
c. die Expression der mindestens zwei Zielgene bestimmt und
d. die Expression der mindestens zwei Zielgene jeweils mit einer Referenzexpression vergleicht, wobei eine Veränderung in der Expression der mindestens zwei Zielgene im Vergleich zu ihrer Referenzexpression ein Hinweis auf eine Veränderung des Immunstatus des Individuums ist und eine Korrelation zwischen der Expression der mindestens zwei Zielgene und ihrer Referenzexpression ein Hinweis auf die Normalität des Immunstatus des Individuums ist.

2. Verfahren nach Anspruch 1, wobei die Blutprobe eine Vollblutprobe ist.

3. Verfahren nach Anspruch 1 oder 2, wobei man in Schritt a. mononukleäre Zellen des peripheren Bluts aus der Blutprobe extrahiert und die anschließenden Schritte b. und d. an diesen mononukleären Zellen des peripheren Bluts durchführt.

4. Verfahren nach einem der Ansprüche 1 bis 3, wobei die für die Expressionsprodukte der Zielgene spezifischen Reagenzien mindestens einen für die Zielgene spezifischen Amplifikationsprimer umfassen.

5. Verfahren nach einem der Ansprüche 1 bis 4, wobei die für die Expressionsprodukte der Zielgene spezifischen Reagenzien mindestens eine für die Zielgene spezifische Hybridisierungssonde umfassen.

6. Verfahren nach einem der Ansprüche 1 bis 5, wobei die für die Expressionsprodukte der Zielgene spezifischen Reagenzien mindestens einen Antikörper umfassen.

7. Verwendung von mindestens zwei Reagenzien, die für mindestens zwei Expressionsprodukte von mindestens zwei Zielgenen spezifisch sind, die jeweils aus den folgenden Zielgenen ausgewählt werden:
HLA-DRA, S100A9, S100A8, IRAK-M, LY64, CIITA, TNF-alpha, IL-10, GBP1, MMP7, CXCL1, CXCL10, COX2, FCN1, TNFAIP6, CXCL7, CXCL5, PID1 und RHOU,
zur Bestimmung des Gesamt-Immunstatus eines septischen Individuums *in vitro,* um eine Therapie umzusetzen, die je nach der festgestellten Immunantwort am stärksten zielgerichtet ist.

8. Verwendung nach Anspruch 7, wobei die für die Expressionsprodukte der Zielgene spezifischen Reagenzien mindestens einen Amplifikationsprimer umfassen.

9. Verwendung nach einem der Ansprüche 7 oder 8, wobei die für die Expressionsprodukte der Zielgene spezifischen Reagenzien mindestens eine Hybridisierungssonde umfassen.

10. Verwendung nach einem der Ansprüche 7 oder 8, wobei die für die Expressionsprodukte der Zielgene spezifischen Reagenzien mindestens einen Antikörper umfassen.

## Claims

1. A process for determining *in vitro* the overall immune status of a septic individual in order to implement the best-targeted therapy according to the established immune response according to which:
a. a blood sample from the individual is provided,
b. at least two reagents specific to at least two products of expression of at least two target genes respectively chosen from the following target genes:
HLA-DRA, S100A9, S100A8, IRAK-M, LY64, CIITA, TNF-alpha, IL-10, GBP1, MMP7, CXCL1, CXCL10, COX2, FCN1, TNFAIP6, CXCL7, CXCL5, PID1 et RHOU,
c. the expression of said at least two target genes is determined, and
d. the expression of said at least two target genes respectively is compared with a reference expression, with a change in the expression of said at least two target genes relative to their reference expression indicating that the individual's immune status has changed, and a correlation between the expression of said at least two target genes with their reference expression indicating that the individual's immune status is normal.

2. The process according to claim 1, wherein the blood sample is a whole blood sample.

3. The process according to claim 1 or 2, wherein in step a. the peripheral blood mononuclear cells are extracted from the blood sample and the subsequent steps b. to d. are carried out on said peripheral blood mononuclear cells.

4. The process according to any one of the claims 1 to 3, wherein the reagents specific to the products of expression of the target genes comprise at least one amplification primer specific to said target genes.

5. The process according to any one of claims 1 to 4, wherein the reagents specific to the products of expression of the target genes comprise at least one hybridisation probe specific to said target genes.

6. The process according to any one of claims 1 to 5, wherein the reagents specific to the products of expression of the target genes comprise at least one antibody.

7. Use of at least two reagents specific to at least two products of expression of at least two target genes respectively chosen from the following target genes:
HLA-DRA, S100A9, S100A8, IRAK-M, LY64, CIITA, TNF-alpha, IL-10, GBP1, MMP7, CXCL1, CXCL10, COX2, FCN1, TNFAIP6, CXCL7, CXCL5, PID1 et RHOU, for determining *in vitro* the overall immune status of a septic individual in order to implement the best-targeted therapy according to the established immune response.

8. The use according to claim 7, wherein the reagents specific to the products of expression of said target genes comprise at least one amplification primer.

9. The use according to claim 7 or 8, wherein the reagents specific to the products of expression of said target genes comprise at least one hybridisation probe.

10. The use according to claim 7 or 8, wherein the reagents specific to the products of expression of said target genes comprise at least one antibody.
